# EUROPEAN PATENT SPECIFICATION

(11) **EP 1 781 697 B1**
(45) Date of publication and mention of the grant of the patent: **22.04.2009**
(21) Application number: 05755533.6
(22) Date of filing: 07.07.2005
(51) Int. Cl.: C07K 14/505, A61K 38/18

(54) **NOVEL CARBAMYLATED EPO AND METHOD FOR ITS PRODUCTION**
NEUES CARBAMYLIERTES EPO UND VERFAHREN ZU DESSEN HERSTELLUNG
NOUVELLE ERYTHROPOIETINE CARBAMYLEE ET SON PROCEDE DE PRODUCTION

(30) Priority: 07.07.2004 DK 200401075; 07.07.2004 US 586370 P; 23.06.2005 US 693870 P
(43) Date of publication of application: 09.05.2007
(73) Proprietor: H. Lundbeck A/S, 2500 Valby-Copenhagen (DK)
(72) Inventor: CHRISTENSEN, Søren, DK-4040 Jyllinge (DK); FOLDAGER, Lars, DK-2970 Hørsholm (DK); VALBJØRN, Jesper, DK-3670 Veksø (DK); THUESEN, Marianne, Hallberg, DK-3520 Farum (DK); PEDERSEN, Anders, Hjelholt, DK-2800 Lyngby (DK); MUNK, Morten, DK-2800 Lyngby (DK)
(74) Representative: Kjerrumgaard, Lars Bo
(86) International application number: PCT/DK2005/000477
(87) International publication number: WO 2006/002646

(56) References cited:
- WO-A-02/053580
- WO-A-20/04112693
- DATABASE CA [Online] CHEMICAL ABSTRACTS SERVICE, COLUMBUS, OHIO, US; PARK, KYUNG-DAE ET AL: "Inhibition of erythropoietin activity by cyanate" XP002346921 retrieved from STN Database accession no. 140:386427 & SCANDINAVIAN JOURNAL OF UROLOGY AND NEPHROLOGY , 38(1), 69-72 CODEN: SJUNAS; ISSN: 0036-5599, 2004,
- DATABASE CA [Online] CHEMICAL ABSTRACTS SERVICE, COLUMBUS, OHIO, US; MUN, KYO-CHEOL ET AL: "Impaired biological activity of erythropoietin by cyanate carbamylation" XP002346922 retrieved from STN Database accession no. 133:130920 & BLOOD PURIFICATION , 18(1), 13-17 CODEN: BLPUDO; ISSN: 0253-5068, 2000,
- LEIST M. ET AL: "Derivatives of Erythropoietin That Are Tissue Protective But Not Erythropoietic" SCIENCE., vol. 305, no. 5681, 9 July 2004 (2004-07-09), pages 239-242, XP002346906 US AMERICAN ASSOCIATION FOR THE ADVANCEMENT OF SCIENCE, WASHINGTON, DC.

## Description

### Introduction

The present invention is directed to a method of producing a carbamylated EPO-compound. The compound, carbamylated erythropoietin (CEPO), which is characterised by being carbamylated on all or most of the primary amines of lysines and on the N-terminal amino acid of the molecule, and in addition this compound has a low level of carbamylation of the primary amines of other amino acids in the molecule. Furthermore, this compound is free of aggregated proteins and polymers, and is suited for use in pharmaceutical compositions for treatment of diseases in for example the central or peripheral nervous system, and other tissues that express the central EPO receptor. One other surprising advantage of the present method of production is the fact that the method provides a product that contains less aggregated protein and less polymers, than the products achieved from other known carbamylation methods described for erythropoietin.

### Background of the invention

The impairment of biological hematopoietic activity of carbamylated EPO has been shown by Satake, R. et al. (1990) Biochimica et Biophysica Acta; 1038: 125-129 and Mun, K-C. and Golper, T.A. (2000) Blood Purif.; 18: 13-17. Brines et al. 2003, US patent application 20030072737 showed that the loss of the hematopoietic activity did not interfere with the tissue protective properties of EPO.

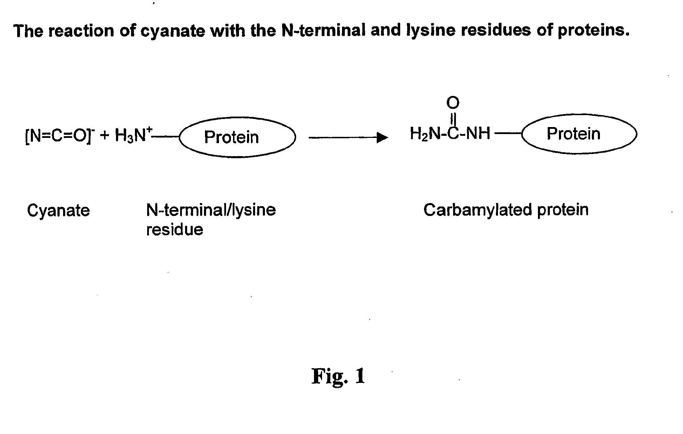

Carbamylation of proteins is widely known as a side effect of using urea in purification of proteins and as a result of high urea serum levels. This is caused by spontaneously decomposition of urea to cyanate. Cyanate is responsible for the carbamylation of the primary amines of the protein hence the N-terminal end and lysines of a protein are susceptible to carbamylation (Figure 1). Additionally other potential amino acid residues susceptible to carbamylation are arginine, cysteine, tyrosine, aspartic acid, glutamic acid and histidine the reaction is however pH dependent and does not proceed as readily as with the N-terminal and lysine residue.

Investigations to reveal if carbamylation of proteins was able to improve or impair the biological activity of proteins have been conducted by Hörkkö, S. et al. (1992) Kidney International.; 41: 1175-1181, Plapp, B.V. et al. (1971) Jour. Biol. Chem.; 246(4): 939-945, Satake, R. et al. (1990) Biochimica et Biophysica Acta; 1038: 125-129 and Mun, K-C. and Golper, T.A. (2000) Blood Purif.; 18: 13-17. They investigated the biological effect of carbamylation of proteins by employing KCNO as the source of cyanate. They all observed a decline or change in the biological activity as a result of increased carbamylation. The assessment of degree of carbamylation was based on two analytical methods:
1. Measurement of the decline in free amino groups using a trinitrobenzenesulfonic acid (TNBS) assay and
2. Amino acid analysis determining the lysines converted to homocitrulline residues.
Hörkkö, S. *et al.* (1992), carbamylated a low-density lipoprotein for the maximum of 6 hours at 37° with 2 M KCNO but did not obtain a fully carbamylated protein as measured with the TNBS assay.

Plapp, B.V. *et al.* (1971), investigated the effect of time and obtained almost a fully carbamylated bovine pancreatic deoxyribonuclease A after a 24 hours treatment with 1 M KCNO at 37°C.

Mun, K-C. and Golper, T.A. (2000) investigated the effect of time with the maximum of 6 hours reaction time using 2 M KCNO. They also investigated the effect of increasing KCNO concentration at 6 hours all reactions were at 37°C. Mun, K-C. and Golper, T.A. (2000) could not, from the experimental design, verify the exact degree of carbamylation (please refer to page 16 line 33-35).

We have now in the present invention found that the carbamylation of EPO yielded polymers and aggregates hence making it unsuitable as a biopharmacutical. In addition we found that the formation of these polymers and aggregates was dependent on the process conditions for the carbamylation. Hence the development of a process with optimal parameters regarding pH, time, cyanate concentration, temperature, protein concentration and most importantly the degree of protein polymerisation was needed. The present invention comprises an optimal process for carbamylation yielding a product with a low degree of polymerisation and aggregation, and furthermore, surprisingly, we have found that a fully carbamylated EPO aiming at the N-terminal and all lysine residues (the latter occurs in a specified pH-range) was obtained. A subsequent step of the method of the invention was made in order to remove the formed aggregates and polymers.

It has previously been illustrated that the degree of carbamylation depends on cyanate concentration and time. However it has not been described how to obtain a scaleable carbamylation process for production of a biopharmaceutical.

The presence of aggregates by sub-optimal production has been associated with the induction of antibodies. And the presence of aggregates therefore results in a biopharmaceutical product unsuitable for use in humans.

The carbamylation and purification process described in the present invention leads to a protein that is characterized as fully carbamylated with the lowest formation of polymers or aggregates as possible and with the minimum loss of end product. Hence making it an economical viable step.

Further processing of the carbamylated protein renders a product useful as a biopharmaceutical with only a minimal risk for the generation of an immunological response to the protein due to aggregates and polymers.

The analytical methods for assessment of full carbamylation are in addition to amino acid analysis; TNBS for free primary amino groups and finally a characterization of the product and digested product by MALDI-TOF.

The resultant compound which is erythropoietin that is fully carbamylated on free amino groups at the N-terminal and lysines of the molecule and further is not aggregated and not polymerised to a content of above 2.5 %, and contains a minimum of over- or under-carbamylated erythropoietin, may be used for the production of pharmaceutical compositions for the treatment of diseases responsive to the neuroprotective effects of native erythropoietin.

### Summary of the invention

The present invention relates to a scaleable protein carbamylation procedure for the production of biopharmaceuticals.

The carbamylation and purification process described in the present application leads to a protein that is characterized as fully carbamylated with the lowest formation of polymers or aggregates as possible and with the minimum loss of end product.

The carbamylation process has been optimised to yield a carbamylated protein with the lowest amount of polymer and aggregates making it an economical viable process. The final product additionally contains limited amounts of isoforms of over- and/or under-carbamylated erythropoietin (below or above 9 carbamylations per molecule). Under-carbamylated EPO would contain less than 9 carbamyl residues, *i*.*e*., not all of the eight lysines and N-terminus are carbamylated. Under-carbamylated EPO can have as a little 5 carbamyl residues and still not have classic erythropoietic activity, making it suitable for use in the present invention. Over-carbamylated EPO has more than 9 carbamyl residues and would have carbamylation at amino acids other than the eight lysine residues and the N-terminus. CEPO can have as many as 15 carbamyl residues and still have the desired effect, *i*.*e*., no classic erythropoietic activity. At least about 90%, and most likely 95%, of the CEPO isoforms are carbamylated at the 8 lysine residues and N-terminus only.

Further processing of the carbamylated protein removes aggregated and polymerised product to a level of maximum 3% or 2.5 %, and thereby renders a product useful as a biopharmaceutical with only minimal risk of generation of an immunological response to the protein due to aggregates and polymers.

The analytical methods for assessment of carbamylation are in addition to amino acid analysis; TNBS for free primary amino groups and a characterization of the product and digested product by MALDI-TOF and LC-MS/MS.

### Detailed description of the invention

### METHOD OF MAKING

Six steps constitute the method for carbamylation of the proteins:
1. Concentration by ultrafiltration
2. Modification by carbamylation.
3. Desalting by gelfiltration
*4. Purification by anion-exchange.*
*5. Concentration and buffer exchange by ultra- and diafiltration.*
*6. 0.22 µm filtration*

The starting material of the present carbamylation process is advantageously purified human EPO, but can be any EPO form of animal or human type, in non-limiting example being it synthetic, recombinant human EPO or biologically or chemically modified human EPO, such as asialo-EPO, mutants of human EPO, *i.e.,* a molecule where changes in the amino acid sequence are introduced, EPO fragments, peptides of EPO, other proteins, or a mixture of proteins if several proteins are desired carbamylated.

The first step of the process involves a protein concentration adjustment by ultrafiltration wherein the protein concentration is adjusted for the purpose of keeping a low process volume. The protein concentration of 0.05-10 mg/ml or 0.05 -8 mg/ml is a preferred embodiment. A more preferred embodiment is 0.05-7 mg/ml and most preferred is 2-5 mg/ml. If the concentration is increased aggregates are increasingly formed. The ultrafiltration is performed by means of a BioMax (Millipore) with a MWCO of 5 kDa. Other filters may be applied. In addition the solubility of the protein may be adjusted by adding stabilizers.

After completion of the concentration step, the protein solution is mixed with K-borate tetra hydrate, K-cyanate, with a pH 7-11 or pH 7-10. In a preferred embodiment, the pH is 8-10, and most preferred 9.0. The temperature ranges from 0°-60°C or 0°-50°C or 0°-40°C or 0°-< 37°C but a preferred embodiment is a temperature interval of 30-34°C preferably 32°C, for a time window of 10 minutes-30 days or 30 minutes-30 days or 1 hour-30 days or 1 hour-20 days or 1 hour-10 days or 1 hour-5 days or 1 hour-2 days or 1 hour-26 hours or 18-26 hours or preferred 22 hours-26 hours, most preferably 24 hours. However these preferred intervals could be changed if other process parameters are changed, *i.e.,* temperature, cyanate concentration and protein concentration.

If the temperature is below the limits the yield will be low as carbamylation will be slow and inefficient. If the temperature limits are exceeded, the yield will be low due to increased aggregation. Another crucial parameter is time as the carbamylation will not be complete if the time is decreased or if time is increased the formation of aggregates are observed hence resulting in lower yield.

Therefore a process with coherent parameters are presented, *i*.*e*., if the temperature is lowered the decreased carbamylation reaction can be compensated for by increasing the cyanate concentration and/or reaction time. Additionally, if reaction time is reduced the decreased carbamylation reaction can be compensated for by increasing temperature and/or cyanate concentration. Finally in a process with reduced cyanate concentration the decreased carbamylation reaction can be compensated for by increasing the reaction time and/or temperature.

Therefore in conclusion one significant change of one crucial parameter (time, temperature, cyanate concentration and protein concentration) would imply a change in one or more of the other crucial parameters in order to obtain a fully carbamylated molecule with low formation of aggregates and polymers.

The concentration of borate buffer may be 0.05-2 M but in a preferred embodiment 0.1-1 M and most preferably 0.5 M as cyanate inherently hydrolyses and polymerizes under uptake of protons and lack of buffer capacity results in a drift of the pH of the solution.

In addition the cyanate concentration is preferred in the range of 0.05-10 M or 0.05-8 M or 0.05-6 M or 0.05-4 M or 0.05-2 M, a preferred embodiment being 0.05-1 M and most preferably 0.5 M.

A concentration of 0.5 M borate buffer is required to control the pH drift caused by proton uptake of the 0.5 M cyanate concentration in use. A process using other salts of cyanate and borate may be employed. Additionally other reaction buffers than borate may be employed, *e*.*g*., a carbonate buffer or phosphate buffer.

The desalting of the reaction mixture of protein and cyanate is performed by means of a chromatographic gelfiltration. The G-25 Fine (Amersham Biosciences) matrice is employed. The hold up time before sample application to the column is controlled and should not exceed 2 hours, as this would cause further carbamylation and polymer formation. The desalting and buffer change of proteins can be performed by dialysis, dia-ultrafiltration or by means of a chromatographic gelfiltration. Other gelfiltration matrices may be applied such as for example matrices of crosslinked polysaccharides or crosslinked mixed polysaccharides, polyacrylamide, polystyrene or matrices of ceramic nature. Furthermore the column height may be varied in this step.

The carbamylation step may be adjusted to obtain a product with less than 40% aggregates and polymers or less than 30% or less than 25% or less than 20 % or less than 15 % or less than 12.5 % or less than 10% or less than 8% or less than 7%.

The removal of aggregates and polymers is performed by a purification step using anion exchange. It is observed that it can separate carbamylated EPO from remains of the starting material and from aggregates/polymers. The running buffer A is: 0.3% Tris (25 mM), 0.3% (50 mM) NaCl. pH 8.5 ± 0.2, and elution buffer B: 0.3% Tris (25 mM), 5.8% (1 M) NaCl. pH 8.5 ± 0.2. The gradient is performed with 0-30 % over 20 column volumes yielding the desired separation. The purification step may result in a product with less than 3 % aggregates and polymers or less than 2.5% or less than 2% or less than 1.5% or less than 1% or less than about 0.5%.

The elution and collection and pooling of the carbamylated EPO peak influence the distribution of the heterogeneity, *i.e*., isoforms, of the eluted protein. In other words, the amounts of over- and under-carbamylated CEPO will vary depending on the collection and pooling procedure. A narrow pooling will lead to a lowering of the content of over- and/or under-carbamylated erythropoietin. Increasing the length of the gradient will allow for selection of a more defined product by leaving out some species.

A composition of carbamylated EPO with less than about 40% over- and under-carbamylated isoforms by weight, as measured bv ESI-mass spectrometry is one result of the invention. A more preferred aspect is a CEPO with less than about 35% of over- and under- carbamylated isoforms by weight, as measured by ESI-mass spectrometry. An even more preferred aspect is a CEPO with less than about 30% over- and under-carbamylated isoforms by weight, as measured by ESI-mass spectrometry. An even more preferred aspect is a CEPO with less than about 25% over- and under-carbamylated isoforms by weight, as measured by ESI-mass spectrometry. An even more preferred aspect is a CEPO with less than about 20% over- and under-carbamylated isoforms by weight, as measured by ESI-mass spectrometry. An even more preferred aspect is a CEPO with less than about 15% over- and under-carbamylated isoforms by weight, as measured by ESI-mass spectrometry. An even more preferred aspect is a CEPO with less than about 10% over- and under-carbamylated isoforms by weight, as measured by ESI-mass spectrometry. An even more preferred aspect is a CEPO with less than about 5% over- and under-carbamylated isoforms by weight, as measured by ESI-mass spectrometry. An even more preferred aspect is a CEPO with less than about 2% over- and under-carbamylated isoforms by weight, as measured by ESI-mass spectrometry. The most preferred aspect is a CEPO with less than about 1% over- and under-carbamylated isoforms by weight, as measured by ESI-mass spectrometry.

In addition to influencing the overall content of the isoforms, the collection and pooling of the carbamylated EPO peak influences the distribution of over-carbamylated CEPO. It is preferred that the amount of over-carbamylated CEPO isoforms be less than about 35% by weight as measured by ESI-mass spectrometry. It is even more preferred that the amount of over-carbamylated CEPO be less than about 30% by weight as measured by ESI-mass spectrometry, and even more preferred that the amount of over-carbamylated CEPO be less than about 25% by weight, and even more preferred that it be less than about 20%, and even more preferred that it be less than about 15%. Most preferably the amount of over-carbamylated EPO should be no more than about 10%, about 5,% or about 1% by weight of the total CEPO.

Other running and elution buffers may be employed as other anion exchange matrices and charged filters may be employed. The matrices in unlimiting example being of crosslinked polysaccharides or crosslinked mixed polysaccharides, polyacrylamide, polystyrene or matrices of ceramic nature.

In addition even cationexchange, hydrophobic interaction chromatography, reversed phase chromatography, affinity chromatography and size exclusion chromatography may be used for the purification.

In the next step for the adjustment of concentration and buffer a dia/ultrafiltration tangential flow filtration unit is used. The carbamylated EPO is adjusted to a concentration > 0.5 mg/ml and the buffer changed to a 20 mM citrate, 100 mM NaCl buffer. The concentration and buffer change is performed by means of a BioMax (Millipore) with a MWCO of 5 kDa. Other filters may be applied.

Finally the purified biopharmaceutical drug substance is 0.22 µm filtrated using a Millipak (Millipore) to reduce germs. Any 0.22 µm filter may be used.

Using the method a fully carbamylated EPO is obtained with less than 3% or preferably less than 2.5% of aggregates as measured by SEC-HPLC. The full carbamylation of the 8 lysine residues is verified using amin oacid analysis determining the converted lysines to homocitrulline. Furthermore the carbamylation was followed using the TNBS assay for determination of primary amines hence showing the complete carbamylation of lysines and the N-terminal.

In addition a thorough characterization using MALDI-TOF determined the change in the intact mass both of the PNGase treated protein and for the protein with the N-glycans. In addition MALDI-TOF peptide mass fingerprint analysis/LC-MS/MS analysis, showed that all 8 lysines and the N-terminal are carbamylated. No other carbamylated amino acids were detected and no modifications of the glycans were detected. Furthermore, a reduced content of over- and under- carbamylated forms of EPO) is obtained in the final product.

One aspect as described herein is the composition obtained after the carbamylation step, but before the anion exchange purification, comprising a carbamylated EPO with less than about 40% by weight of aggregates and polymers, or less than about 30%, or less than about 25%, or less than about 20 %, or less than about 15 %, or less than about 12.5 %, or less than about 10%, or less than about 8% or less than about 7%, and an amount of cyanate.

One further aspect is the composition obtained after the anion exchange purification comprising a carbamylated EPO with less than about 3% by weight of aggregates and polymers, or less than about 2.5%, or less than about 2%, or less than about 1.5%, or less than about 1% or less than about 0.5%. Further, this composition comprises isoforms consisting of over- or under-carbamylated EPO in amounts less than about 40 % by weight of the total carbamylated EPO, or more preferably less than about 35%, or less than about 30%, or less than about 25%, or less than about 20%, or less than about 15%, or less than about 10%, or less than about 7.5%, or less than about 5%, or less than about 2%, and most prefereably less than about 1%. Further, the amount of over-carbamylated EPO in the composition may be less than about 35% by weight of the total carbamylated EPO, or more preferably less than about 30%, or less than about 25%, or less than about 20%, or less than about 15%,, or less than about 10%, or less than about 7.5%, or less than about 5%, or less than about 2%, and most preferably less than about 1%.

### PHARMACEUTICAL COMPOSITIONS

One aspect is the use of the compounds as described herein, resulting from the inventive method for the production of pharmaceutical compositions to be used in humans or mammals for treatment of the conditions described below.

One aspect is a pharmaceutical composition comprising a therapeutically effective amount of carbamylated EPO, with less than about 3% by weight of aggregates and polymers, or more preferably less than about 2.5%, or less than about 2%, or less than about 1.5%, or less than about 1%, and most preferably, less than about 0.5% and further, this composition comprises isoforms consisting of over- or under- carbamylated EPO in amounts less than about 40 % by weight of total carbamylated EPO, or more preferably less than about 35%, or less than about 30%, or less than about 25%, or less than about 20 %, or less than about 15%, or less than about 10%, or less than about 5 %, or less than about 3%, or less than about 2% , and most preferably less than about 1%. Further, the amount of over-carbamylated EPO in the composition may be less than about 35% by weight of the total carbamylated EPO, or more preferably less than about 30%, or less than about 25%, or less than about 20%, or less than about 15%, or less than about 10%, or less than about 5%, or less than about 3%, or less than about 2% and most preferably less than about 1%.

In the practice of one aspect, a pharmaceutical composition as described above containing the compound as described herein, resulting from the inventive method may be administerable to a mammal by any route that provides a sufficient level of the compound as described herein, resulting from the inventive method in the vasculature to permit translocation across an endothelial cell barrier and beneficial effects on responsive cells. When used for the purpose of perfusing a tissue or organ, similar results are desired. In the instance where the cells or tissue is non-vascularized and/or the administration is by bathing the cells or tissue with the composition, the pharmaceutical composition provides an effective responsive cell-beneficial amount of a compound as described herein, resulting from the inventive method. The endothelial cell barriers across which the compound as described herein, resulting from the inventive method may translocate include tight junctions, perforated junctions, fenestrated junctions, and any other types of endothelial barriers present in a mammal. A preferred barrier is an endothelial cell tight junction.

The aforementioned compound as described herein, resulting from the inventive method is useful generally for the therapeutic or prophylactic treatment of human diseases of the central nervous system or peripheral nervous system which have primarily neurological or psychiatric symptoms, ophthalmic diseases, cardiovascular diseases, cardiopulmonary diseases, respiratory diseases, kidney, urinary and reproductive diseases, gastrointestinal diseases and endocrine and metabolic abnormalities. In particular, such conditions and diseases include hypoxic conditions, which adversely affect excitable tissues, such as excitable tissues in the central nervous system tissue, peripheral nervous system tissue, or cardiac or retinal tissue such as, for example, brain, heart, or retina/eye. Therefore, the compound as described herein, resulting from the inventive method can be used to treat or prevent damage to excitable tissue resulting from hypoxic conditions in a variety of conditions and circumstances. Non-limiting examples of such conditions and circumstances are provided in the table hereinbelow.

In the example of the protection of neuronal tissue pathologies treatable as described herein, such pathologies include those resulting from reduced oxygenation of neuronal tissues. Any condition which reduces the availability of oxygen to neuronal tissue, resulting in stress, damage, and finally, neuronal cell death, can be treated by the presently described methods. Generally referred to as hypoxia and/or ischemia, these conditions arise from or include, but are not limited to, stroke, vascular occlusion, prenatal or postnatal oxygen deprivation, suffocation, choking, near drowning, carbon monoxide poisoning, smoke inhalation, trauma, including surgery and radiotherapy, asphyxia, epilepsy, hypoglycemia, chronic obstructive pulmonary disease, emphysema, adult respiratory distress syndrome, hypotensive shock, septic shock, anaphylactic shock, insulin shock, sickle cell crisis, cardiac arrest, dysrhythmia, nitrogen narcosis, and neurological deficits caused by heart-lung bypass procedures.

In one aspect, for example, the specific pharmaceutical compositions comprising the composition can be administered to prevent injury or tissue damage resulting from risk of injury or tissue damage during surgical procedures, such as, for example, tumor resection or aneurysm repair. Other pathologies caused by or resulting from hypoglycemia which are treatable by the methods described herein include insulin overdose, also referred to as iatrogenic hyperinsulinemia, insulinoma, growth hormone deficiency, hypocortisolism, drug overdose, and certain tumors.

Other pathologies resulting from excitable neuronal tissue damage include seizure disorders, such as epilepsy, convulsions, or chronic seizure disorders. Other treatable conditions and diseases include, but are not limited to, diseases such as stroke (ischemic stroke, subarachnoid haemorrhage, Intracerebral haemorrhage), multiple sclerosis, hypotension, cardiac arrest, Alzheimer's disease, Parkinson's disease, cerebral palsy, brain or spinal cord trauma, AIDS dementia, age-related loss of cognitive function, memory loss, amyotrophic lateral sclerosis, seizure disorders, alcoholism, retinal ischemia, optic nerve damage resulting from glaucoma, and neuronal loss.

The specific composition as described herein and methods of the present invention may be used to treat inflammation resulting from disease conditions or various traumas, such as physically or chemically induced inflammation. Such traumas could include angitis, chronic bronchitis, pancreatitis, osteomyelitis, rheumatoid arthritis, glomerulonephritis, optic neuritis, temporal arteritis, encephalitis, meningitis, transverse myelitis, dermatomyositis, polymyositis, necrotizing fascilitis, hepatitis, and necrotizing enterocolitis.

Evidence has demonstrated that activated astrocytes can exert a cytotoxic role towards neurons by producing neurotoxins. Nitric oxide, reactive oxygen species, and cytokines are released from glial cells in response to cerebral ischemia (see Becker, K.J. 2001. Targeting the central nervous system inflammatory response in ischemic stroke. Curr Opinion Neurol 14:349-353 and Mattson, M.P., Culmsee, C., and Yu, Z.F. 2000. Apoptotic and Antiapoptotic mechanisms in stroke. Cell TissueRes 301:173-187.). Studies have further demonstrated that in models of neurodegeneration, glial activation and subsequent production of inflammatory cytokines depends upon primary neuronal damage (see Viviani, B., Corsini, E., Galli, C.L., Padovani, A., Ciusani, E., and Marinovich, M. 2000. Dying neural cells activate glia through the release of a protease product. Glia 32:84-90 and Rabuffetti, M., Scioratti, C., Tarozzo, G., Clementi, E., Manfredi, A.A., and Beltramo, M. 2000. Inhibition of caspase-1-like activity by Ac-Tyr-Val-Ala-Asp-chloromethyl ketone includes long lasting neuroprotection in cerebral ischemia through apoptosis reduction and decrease of proinflammatory cytokines. J Neurosci 20:4398-4404). Inflammation and glial activation is common to different forms of neuro degenerative disorders, including cerebral ischemia, brain trauma and experimental allergic encephalomyelitis, disorders in which erythropoietin exerts a neuroprotective effect. Inhibition of cytokine production by erythropoietin could, at least in part, mediate its protective effect. However, unlike "classical" anti-inflammatory cytokines such as Il-10 and IL-13, which inhibit tumor necrosis factor production directly, erythropoietin appears to be active only in the presence of neuronal death.

While not wishing to be bound by any particular theory, it appears that this anti-inflammatory activity may be hypothetically explained by several non-limiting theories. First, since erythropoietin prevents apoptosis, inflammatory events triggered by apoptosis would be prevented. Additionally, erythropoietin may prevent the release of molecular signals from dying neurons which stimulate the glia cells or could act directly on the glial cells reducing their reaction to these products. Another possibility is that erythropoietin targets more proximal members of the inflammatory cascade (*e*.*g*., caspase 1, reactive oxygen or nitrogen intermediates) that trigger both apoptosis and inflammation.

Furthermore, erythropoietin appears to provide anti-inflammatory protection without the rebound affect typically associated with other anti-inflammatory compounds such as dexamethasone. Once again, not wishing to be bound by any particular theory, it appears as though this may be due to erythropoietin's affect on multipurpose neuro toxins such as nitric oxide (NO). Although activated astrocytes and microglia produce neurotoxic quantities of NO in response to various traumas, NO serves many purposes within the body including the modulation of essential physiological functions. Thus, although the use of an antiinflammatory may alleviate inflammation by suppressing NO or other neuro toxins, if the anti-inflammatory has too long a half-life it may also interfere with these chemicals' roles in repairing the damage resulting from the trauma that led to the inflammation. It is hypothesized that the compound of the present invention is able to alleviate the inflammation without interfering with the restorative capabilities of neurotoxins such as NO.

The specific compositions and methods may be used to treat conditions of, and damage to, retinal tissue. Such disorders include, but are not limited to retinal ischemia, macular degeneration, retinal detachment, retinitis pigmentosa, arteriosclerotic retinopathy, hypertensive retinopathy, retinal artery blockage, retinal vein blockage, hypotension, and diabetic retinopathy.

In another embodiment, the methods and principles may be used to protect or treat injury resulting from radiation damage or chemotherapy induced damage to excitable tissue. In nonlimiting example, to protect from damages caused by compounds like taxanes, cisplatin and other chemotherapeutics with the potential to induce peripheral neuropathies. A further utility of the methods is in the treatment of neurotoxin poisoning, such as domoic acid shellfish poisoning, neurolathyrism, and Guam disease, amyotrophic lateral sclerosis, and Parkinson's disease.

As mentioned above, also described is a method for enhancing excitable tissue function in a mammal by peripheral administration of a compound as described above. Various diseases and conditions are amenable to treatment using this method, and further, this method is useful for enhancing cognitive function in the absence of any condition or disease. These uses described in further detail below and include enhancement of learning and training in both human and non-human mammals.

Conditions and diseases treatable by the methods of this aspect directed to the central nervous system include, but are not limited to, mood disorders, anxiety disorders, depression, autism, attention deficit hyperactivity disorder, and cognitive dysfunction. These conditions benefit from enhancement of neuronal function. Other disorders treatable in accordance with the present teachings include for example, sleep disruption, sleep apnea, and travel-related disorders; subarachnoid and aneurismal bleeds, hypotensive shock, concussive injury, septic shock, anaphylactic shock, and sequelae of various encephalitides and meningitides, for example, connective tissue disease-related cerebritides such as lupus. Other uses include prevention of or protection from poisoning by neurotoxins, such as domoic acid shellfish poisoning, neurolathyrism, and Guam disease, amyotrophic lateral sclerosis, Parkinson's disease; postoperative treatment for embolic or ischemic injury; whole brain irradiation; sickle cell crisis; and eclampsia.

A further group of conditions treatable by the methods include mitochondrial dysfunction, of either a hereditary or an acquired nature, which are the cause of a variety of neurological diseases typified by neuronal injury and death. For example, Leigh disease (subacute necrotizing encephalopathy) is characterized by progressive visual loss and encephalopathy, due to neuronal drop out, and myopathy. In these cases, defective mitochondrial metabolism fails to supply enough high energy substrates to fuel the metabolism of excitable cells. An erythropoietin receptor activity modulator optimizes failing function in a variety of mitochondrial diseases. As mentioned above, hypoxic conditions adversely affect excitable tissues. The excitable tissues include, but are not limited to, central nervous system tissue, peripheral nervous system tissue, and heart tissue. In addition to the conditions described above, the methods are useful in the treatment of inhalation poisoning, such as carbon monoxide and smoke inhalation, severe asthma, adult respiratory distress syndrome, choking, and near drowning. Further conditions which create hypoxic conditions or by other means induce excitable tissue damage include hypoglycemia that may occur in inappropriate dosing of insulin, or with insulin-producing neoplasms (insulinoma).

Various neuropsychologic disorders which are believed to originate from excitable tissue damage are treatable by the instant methods. Chronic disorders in which neuronal damage is involved and for which treatment is provided include disorders relating to the central nervous system and/or peripheral nervous system including age-related loss of cognitive function and senile dementia, chronic seizure disorders, Alzheimer's disease, Parkinson's disease, dementia, memory loss, amyotrophic lateral sclerosis, multiple sclerosis, tuberous sclerosis, Wilson's Disease cerebral and progressive *supra*nuclear palsy, Guam disease, Lewy body dementia, prion diseases, such as spongiform encephalopathies, *e.g.,* Creutzfeldt-Jakob disease, Huntington's disease, myotonic dystrophy, Charcot-Marie-Tooth Disease, Freidrich's ataxia and other ataxias, as well as Gilles de la Tourette's syndrome, seizure disorders such as epilepsy and chronic seizure disorder, stroke, brain or spinal cord trauma, AIDS dementia, alcoholism, autism, retinal ischemia, glaucoma, autonomic function disorders such as hypertension and sleep disorders, and neuropsychiatric disorders that include, but are not limited to, schizophrenia, schizoaffective disorder, attention deficit disorder hyperactivity, dysthymic disorder, major depressive disorder, mania, obsessive-compulsive disorder, psychoactive substance use disorders, anxiety, panic disorder, as well as unipolar and bipolar affective disorders. Additional neuropsychiatric and neurodegenerative disorders include, for example, those listed in the American Psychiatric Association's Diagnostic and Statistical Manual of Mental Disorders (DSM), the most current version, IV, of which in incorporated herein by reference in its entirety.

In another aspect, recombinant chimeric toxin molecules comprising a compound as described herein, resulting from the inventive method can be used for therapeutic delivery of toxins to treat a proliferative disorder, such as cancer, or viral disorder, such as subacute sclerosing panencephalitis.

Table 1 lists additional exemplary, non-limiting indications as to the various conditions and diseases amenable to treatment by the aforementioned compounds as described herein, resulting from the inventive method.

**Table 1**

| ***Cell, tissue or organ*** | ***Dysfunction or pathology*** | ***Condition or disease*** | ***Type*** |
|---|---|---|---|
| Heart | Ischemia | Coronary artery disease | Acute, chronic Stable, unstable |
| | | Myocardial infarction | Dressler's syndrome |
| | | Angina | |
| | | Congenital heart disease | Valvular Cardiomyopathy |
| | | Prinzmetal angina | |
| | | Cardiac rupture | Aneurysmatic Septal perforation |
| | | Angiitis | |
| | Arrhythmia | Tachy-, bradyarrhythmia Supraventricular, ventricular Conduction abnormalities | Stable, unstable Hypersensitive carotid sinus node |
| | Congestive heart failure | Left, right, bi-ventricular, systolic, diastolic | Cardiomyopathies, such as idiopathic familial, infective, metabolic, storage disease, deficiencies, connective tissue disorder, infiltration and granulomas, neurovascular |
| | | Myocarditis | Autoimmune, infective, idiopathic |
| | | Cor pulmonale | |
| | Blunt and penetrating trauma | | |
| | Toxins | Cocaine toxicity | |
| Vascular | Hypertension | Primary, secondary | |
| | Decompression sickness | | |
| | Fibromuscular hyperplasia | | |
| | Aneurysm | Dissecting, ruptured, enlarging | |
| Lungs | Obstructive | Asthma Chronic bronchitis, Emphysema and airway obstruction | |
| | Ischemic lung disease | Pulmonary embolism, Pulmonary thrombosis, Fat embolism | |
| | Environmental lung diseases | | |
| | Ischemic lung disease | Pulmonary embolism Pulmonary thrombosis | |
| | Interstitial lung disease | Idiopathic pulmonary fibrosis | |
| | Congenital | Cystic fibrosis | |
| | Cor pulmonale | | |
| | Trauma | | |
| | Pneumonia and pneumonitides | Infectious, parasitic, toxic, traumatic, bum, aspiration | |
| | Sarcoidosis | | |
| Pancreas | Endocrine | Diabetes mellitus, type I and II | Beta cell failure, dysfunction Diabetic neuropathy |
| | | Other endocrine cell failure of the pancreas | |
| | Exocrine | Exocrine pancreas failure | pancreatitis |
| Bone | Osteopenia | Primary Secondary | Hypogonadism immobilisation Postmenopausal Age-related Hyperparathyroidism Hyperthyroidism Calcium, magnesium, phosphorus and/or vitamin D deficiency |
| | Osteomyelitis | | |
| | Avascular necrosis | | |
| | Trauma | | |
| | Paget's disease | | |
| Skin | Alopecia | Areata Totalis | Primary Secondary Male pattern baldness |
| | Vitiligo | Localized generalized | Primary secondary |
| | Diabetic ulceration | | |
| | Peripheral vascular disease | | |
| | Bum injuries | | |
| Autoimmune disorders | Lupus erythematodes, Sjiogren, Rheumatoid arthritis, Glomerulonephritis, Angiitis | | |
| | Langerhan's histiocytosis | | |
| Eye | Optic neuritis | | |
| | Blunt and penetrating injuries, Infections, Sarcoid, Sickle C disease, Retinal detachment, Temporal arteritis | | |
| | Retinal ischemia, Macular degeneration, Retinitis pigmentosa, Arteriosclerotic retinopathy, Hypertensive retinopathy, Retinal artery blockage, Retinal vein blockage, Hypotension, Diabetic retinopathy, and Macular edema | | |
| Embryonic and fetal disorders | Asphyxia | | |
| | Ischemia | | |
| CNS | Chronic fatigue syndrome, acute and chronic hypoosmolar and hyperosmolar syndromes, AIDS Dementia, Electrocution | | |
| | Encephalitis | Rabies, Herpes | |
| | Meningitis | | |
| | Subdural hematoma | | |
| | Nicotine addiction | | |
| | Drug abuse and withdrawal | Cocaine, heroin, crack, marijuana, LSD, PCP, poly-drug abuse, ecstasy, opioids, sedative hypnotics, amphetamines, caffeine | |
| | Obsessive-compulsive disorders | | |
| | Spinal stenosis, Transverse myelitis, Guillian Barre, Trauma, Nerve root compression, Tumoral compression, Heat stroke | | |
| ENT | Tinnitus Meuniere's syndrome Hearing loss | | |
| | Traumatic injury, barotraumas | | |
| Kidney | Renal failure | Acute, chronic | Vascular/ischemic, interstitial disease, diabetic kidney disease, nephrotic syndromes, infections, injury, contrast-induced, chemotherapy-induced, CPB-induced, or preventive |
| | Henoch S. Purpura | | |
| Striated muscle | Autoimmune disorders | Myasthenia gravis Dermatomyositis Polymyositis | |
| | Myopathies | Inherited metabolic, endocrine and toxic | |
| | Heat stroke | | |
| | Crush injury | | |
| | Rhabdomylosis | | |
| | Mitochondrial disease | | |
| | Infection | Necrotizing fasciitis | |
| Sexual dysfunction | Central and peripheral (e.g. erectile dysfunction) | Impotence secondary to medication, (diabetes) | |
| Liver | Hepatitis | Viral, bacterial, parasitic | |
| | Ischemic disease | | |
| | Cirrhosis, fatty liver | | |
| | Infiltrative/metabolic diseases | | |
| Gastrointestinal | Ischemic bowel disease | | |
| | Inflammatory bowel disease | | |
| | Necrotizing enterocolitis | | |
| Organ transplantation | Treatment of donor and recipient | | |
| Reproductive tract | Infertility | Vascular Autoimmune Uterine abnormalities Implantation disorders | |
| Endocrine | Glandular hyper- and hypofunction | | |

As mentioned above, these diseases, disorders or conditions are merely illustrative of the range of benefits provided by the present compound. Accordingly, described herein are generally therapeutic or prophylactic treatment of the consequences of mechanical trauma or of human diseases. Therapeutic or prophylactic treatment for diseases, disorders or conditions of the CNS and/or peripheral nervous system are preferred. Therapeutic or prophylactic treatment for diseases, disorders or conditions which have a psychiatric component is provided. Therapeutic or prophylactic treatment for diseases, disorders or conditions including, but not limited to, those having an ophthalmic, cardiovascular, cardiopulmonary, respiratory, kidney, urinary, reproductive, gastrointestinal, endocrine, or metabolic component is provided.

In one aspect such a pharmaceutical composition comprising the compound as described herein, resulting from the inventive method may be administered systemically to protect or enhance the target cells, tissue, or organ. Such administration may be parenterally, via inhalation, or transmucosally, *e*.*g*., orally, nasally, rectally, intravaginally, sublingually, submucosally or transdermally. Preferably, administration is parenteral, *e*.*g*., via intravenous or intraperitoneal injection, and also including, but is not limited to, intra-arterial, intramuscular, intradermal and subcutaneous administration.

For other routes of administration, such as by use of a perfusate, injection into an organ, or other local administration, a pharmaceutical composition will be provided which results in similar levels of the compound as described herein, resulting from the inventive method. A level of about 0.01pM -30 nM is preferred.

The pharmaceutical compositions as described herein, resulting from the inventive method may comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized foreign pharmacopeia for use in animals, and more particularly in humans. The term "carrier" refers to a diluent, adjuvant, excipient, or vehicle with which the therapeutic is administered. Such pharmaceutical carriers can be sterile liquids, such as saline solutions in water and oils, including those of petroleum, animal, vegetable or synthetic origin, such as peanut oil, soybean oil, mineral oil, sesame oil and the like. A saline solution is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. The compounds of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the patient. The formulation should suit the mode of administration.

Pharmaceutical compositions adapted for oral administration may be provided as capsules or tablets; as powders or granules; as solutions, syrups or suspensions (in aqueous or nonaqueous liquids); as edible foams or whips; or as emulsions. Tablets or hard gelatine capsules may comprise lactose, starch or derivatives thereof, magnesium stearate, sodium saccharine, cellulose, magnesium carbonate, stearic acid or salts thereof. Soft gelatine capsules may comprise vegetable oils, waxes, fats, semi-solid, or liquid polyols etc. Solutions and syrups may comprise water, polyols, and sugars.

An active agent intended for oral administration may be coated with or admixed with a material that delays disintegration and/or absorption of the active agent in the gastrointestinal tract (*e*.*g*., glyceryl monostearate or glyceryl distearate may be used). Thus, the sustained release of an active agent may be achieved over many hours and, if necessary, the active agent can be protected from being degraded within the stomach. Pharmaceutical compositions for oral administration may be formulated to facilitate release of an active agent at a particular gastrointestinal location due to specific pH or enzymatic conditions.

Pharmaceutical compositions adapted for transdermal administration may be provided as discrete patches intended to remain in intimate contact with the epidermis of the recipient for a prolonged period of time. Pharmaceutical compositions adapted for topical administration may be provided as ointments, creams, suspensions, lotions, powders, solutions, pastes, gels, sprays, aerosols or oils. For topical administration to the skin, mouth, eye or other external tissues a topical ointment or cream is preferably used. When formulated in an ointment, the active ingredient may be employed with either a paraffinic or a water-miscible ointment base. Alternatively, the active ingredient may be formulated in a cream with an oil-in-water base or a water-in-oil base. Pharmaceutical compositions adapted for topical administration to the eye include eye drops. In these compositions, the active ingredient can be dissolved or suspended in a suitable carrier, *e*.*g*., in an aqueous solvent. Pharmaceutical compositions adapted for topical administration in the mouth include lozenges, pastilles, and mouthwashes.

Pharmaceutical compositions adapted for nasal and pulmonary administration may comprise solid carriers such as powders (preferably having a particle size in the range of 20 to 500 microns). Powders can be administered in the manner in which snuff is taken, *i.e.,* by rapid inhalation through the nose from a container of powder held close to the nose. Alternatively, compositions adopted for nasal administration may comprise liquid carriers, *e*.*g*., nasal sprays or nasal drops. Alternatively, inhalation directly into the lungs may be accomplished by inhalation deeply or installation through a mouthpiece into the oropharynx. These compositions may comprise aqueous or oil solutions of the active ingredient. Compositions for administration by inhalation may be supplied in specially adapted devices including, but not limited to, pressurized aerosols, nebulizers or insufflators, which can be constructed so as to provide predetermined dosages of the active ingredient. In a preferred aspect, pharmaceutical compositions are administered into the nasal cavity directly or into the lungs via the nasal cavity or oropharynx.

Pharmaceutical compositions adapted for rectal administration may be provided as suppositories or enemas. Pharmaceutical compositions adapted for vaginal administration may be provided as pessaries, tampons, creams, gels, pastes, foams or spray formulations.

Pharmaceutical compositions adapted for parenteral administration include aqueous and nonaqueous sterile injectable solutions or suspensions, which may contain antioxidants, buffers, bacteriostats, and solutes that render the compositions substantially isotonic with the blood of an intended recipient. Other components that may be present in such compositions include water, alcohols, polyols, glycerine and vegetable oils, for example. Compositions adapted for parenteral administration may be presented in unit-dose or multi-dose containers, for example sealed ampules and vials, and may be stored in a freeze-dried (lyophilized) condition requiring only the addition of a sterile liquid carrier, *e*.*g*., sterile saline solution for injections, immediately prior to use. Extemporaneous injection solutions and suspensions may be prepared from sterile powders, granules, and tablets. In one aspect, an autoinjector comprising an injectable solution of a compound as described herein, resulting from the inventive method may be provided for emergency use by ambulances, emergency rooms, and battlefield situations, and even for self-administration in a domestic setting, particularly where the possibility of traumatic amputation may occur, such as by imprudent use of a lawn mower. The likelihood that cells and tissues in a severed foot or toe will survive after reattachment may be increased by administering a compound as described herein, resulting from the inventive method to multiple sites in the severed part as soon as practicable, even before the arrival of medical personnel on site, or arrival of the afflicted individual with severed toe at the emergency room.

In a preferred aspect, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically-sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile saline can be provided so that the ingredients may be mixed prior to administration.

Suppositories generally contain active ingredient in the range of 0.5% to 10% by weight; oral formulations preferably contain 10% to 95% active ingredient.

A perfusate composition may be provided for use in transplanted organ baths, for *in situ* perfusion, or for administration to the vasculature of an organ donor prior to organ harvesting. Such pharmaceutical compositions may comprise levels of the compound as described herein, resulting from the inventive method not suitable for acute or chronic, local or systemic administration to an individual, but will serve the functions intended herein in a cadaver, organ bath, organ perfusate, or *in situ* perfusate prior to removing or reducing the levels of the compound as described herein, resulting from the inventive method contained therein before exposing or returning the treated organ or tissue to regular circulation.

Also described is a pharmaceutical pack or kit comprising one or more containers filled with one or more of the ingredients of the pharmaceutical compositions. Optionally associated with such container(s) can be a notice in the form prescribed by a governmental agency regulating the manufacture, use or sale of pharmaceuticals or biological products, which notice reflects approval by the agency of manufacture, use, or sale for human administration.

In another aspect, for example, the compound as described herein, resulting from the inventive method can be delivered in a controlled-release system. For example, the polypeptide may be administered using intravenous infusion, an implantable osmotic pump, a transdermal patch, liposomes, or other modes of administration. In one aspect, a pump may be used (see Langer, *supra*; Sefton, 1987, CRC Crit. Ref. Biomed. Eng. 14:201; Buchwald et al., 1980, Surgery 88:507; Saudek et al., 1989, N. Engl. J. Med. 321:574). In another aspect, the compound can be delivered in a vesicle, in particular a liposome (see Langer, Science 249:1527-1533 (1990); Treat et al., in Liposomes in the Therapy of Infectious Disease and Cancer, Lope-Berestein and Fidler (eds.), Liss, New York, pp. 353-365 (1989); WO 91/04014; U.S. Patent No. 4,704,355; Lopez-Berestein, *ibid*., pp. 317-327; see generally *ibid*.). In another embodiment, polymeric materials can be used (see Medical Applications of Controlled Release, Langer and Wise (eds.), CRC Press: Boca Raton, Florida, 1974; Controlled Drug Bioavailability, Drug Product Design and Performance, Smolen and Ball (eds.), Wiley: New York (1984); Ranger and Peppas, J. Macromol. Sci. Rev. Macromol. Chem. 23:61, 1953; see also Levy et al., 1985, Science 228:190; During et al., 1989, Ann. Neurol. 25:351; Howard et al., 1989, J. Neurosurg. 71:105).

In yet another aspect, a controlled release system can be placed in proximity of the therapeutic target, *i.e.,* the target cells, tissue or organ, thus requiring only a fraction of the systemic dose (see, *e.g.,* Goodson, pp. 115-138 in Medical Applications of Controlled Release, vol. 2, supra, 1984). Other controlled release systems are discussed in the review by Langer (1990, Science 249:1527-1533).

In another aspect, the compound as described herein, resulting from the inventive method, as properly formulated, can be administered by nasal, oral, rectal, vaginal, or sublingual administration.

In a specific aspect, it may be desirable to administer the compositions locally to the area in need of treatment; this may be achieved by, for example, and not by way of limitation, local infusion during surgery, topical application, *e*.*g*., in conjunction with a wound dressing after surgery, by injection, by means of a catheter, by means of a suppository, or by means of an implant, said implant being of a porous, non-porous, or gelatinous material, including membranes, such as silastic membranes, or fibers.

Selection of the preferred effective dose will be determined by a skilled artisan based upon considering several factors which will be known to one of ordinary skill in the art. Such factors include the particular form of compound as described herein, resulting from the inventive method, and its pharmacokinetic parameters such as bioavailability, metabolism, half-life, etc., which will have been established during the usual development procedures typically employed in obtaining regulatory approval for a pharmaceutical compound. Further factors in considering the dose include the condition or disease to be treated or the benefit to be achieved in a normal individual, the body mass of the patient, the route of administration, whether administration is acute or chronic, concomitant medications, and other factors well known to affect the efficacy of administered pharmaceutical agents. Thus the precise dosage should be decided according to the judgment of the practitioner and each patient's circumstances, *e*.*g*., depending upon the condition and the immune status of the individual patient, and according to standard clinical techniques.

In another aspect, a perfusate or perfusion solution is provided for perfusion and storage of organs for transplant, the perfusion solution including an amount of the compound as described herein, resulting from the inventive method, effective to protect responsive cells and associated cells, tissues, or organs. Transplant includes, but is not limited to, xenotransplantation, where a organ (including cells, tissue or other bodily part) is harvested from one donor and transplanted into a different recipient; and autotransplant, where the organ is taken from one part of a body and replaced at another, including bench surgical procedures, in which an organ may be removed, and while ex vivo, resected, repaired, or otherwise manipulated, such as for tumor removal, and then returned to the original location. In one aspect, the perfusion solution is the University of Wisconsin (UW) solution (U.S. Patent No. 4,798,824) which contains from about 1 to about 25 U/ml erythropoietin, 5% hydroxyethyl starch (having a molecular weight of from about 200,000 to about 300,000 and substantially free of ethylene glycol, ethylene chlorohydrin, sodium chloride and acetone); 25mM KH₂PO₄; 3mM glutathione; 5mM adenosine; 10mM glucose; 10mM HEPES buffer; 5mM magnesium gluconate; 1.5mM CaCl2; 10mM sodium gluconate; 200,000 units penicillin; 40 units insulin; 16mg, Dexamethasone; 12mg Phenol Red; and has a pH of 7.4-7.5 and an osmolality of about 320 mOSm/l. The solution is used to maintain cadaveric kidneys and pancreases prior to transplant. Using the solution, preservation can be extended beyond the 30-hour limit recommended for cadaveric kidney preservation. This particular perfusate is merely illustrative of a number of such solutions that can be adapted for the present use by inclusion of an effective amount of the compound as described herein, resulting from the inventive method. In a further aspect, the perfusate solution contains from about 0.01pg/ml to about 400 ng/ml of the compound of the invention, or from about 40 to about 300 ng/ml of the compound as described herein, resulting from the inventive method.

While the preferred recipient of a compound as described herein, resulting from the inventive method for the purposes herein throughout is a human, the methods herein apply equally to other mammals, particularly domesticated animals, livestock, companion and zoo animals. However, the invention is not so limiting and the benefits can be applied to any mammal.

### THERAPEUTIC AND PREVENTATIVE USES OF THE PRESENT COMPOUNDS

As noted in Example 1 below, the presence of erythropoietin preceptors on the brain capillary human endothelium indicates that the targets of the compounds as described herein, resulting from the inventive method are present in the human brain, and that the animal studies on these compounds as described herein, resulting from the inventive method are directly translatable to the treatment or prophylaxis of human beings.

In another aspect, methods and compositions for enhancing the viability of cells, tissues, or organs which are not isolated from the vasculature by an endothelial cell barrier are provided by exposing the cells, tissue or organs directly to a pharmaceutical composition comprising a compound as described herein, resulting from the inventive method, or administering or contacting a compound as described herein, resulting from the inventive method containing pharmaceutical composition to the vasculature of the tissue or organ. Enhanced activity of responsive cells in the treated tissue or organ is responsible for the positive effects exerted.

As described above, the present description is based, in part, on the discovery that erythropoietin molecules can be transported from the luminal surface to the basement membrane surface of endothelial cells of the capillaries of organs with endothelial cell tight junctions, including, for example, the brain, retina, and testis. Thus, responsive cells across the barrier are susceptible targets for the beneficial effects of a compound as described herein, resulting from the inventive method and others cell types or tissues or organs that contain and depend in whole or in part on responsive cells therein are targets for the present methods. While not wishing to be bound by any particular theory, after transcytosis of a compound as described herein, resulting from the inventive method, the compound as described herein, resulting from the inventive method can interact with an erythropoietin receptor on an responsive cell, for example, neuronal, retinal, muscle, heart, lung, liver, kidney, small intestine, adrenal cortex, adrenal medulla, capillary endothelial, testes, ovary, pancreas, bone, skin, or endometrial cell, and receptor binding can initiate a signal transduction cascade resulting in the activation of a gene expression program within the responsive cell or tissue, resulting in the protection of the cell or tissue, or organ, from damage, such as by toxins, chemotherapeutic agents, radiation therapy, hypoxia, etc. Thus, methods for protecting responsive cell-containing tissue from injury or hypoxic stress, and enhancing the function of such tissue are described in detail herein below. As noted above, the methods are equally applicable to humans as well as to other animals.

In the practice of one aspect a mammalian patient is undergoing systemic chemotherapy for cancer treatment, including radiation therapy, which commonly has adverse effects such as nerve, lung, heart, ovarian, or testicular damage. Administration of a pharmaceutical composition comprising a compound as described herein, resulting from the inventive method as described above is performed prior to and during chemotherapy and/or radiation therapy, to protect various tissues and organs from damage by the chemotherapeutic agent, such as to protect the testes. Treatment may be continued until circulating levels of the chemotherapeutic agent have fallen below a level of potential danger to the mammalian body.

In the practice of another aspect, various organs were planned to be harvested from a victim of an automobile accident for transplant into a number of recipients, some of which required transport for an extended distance and period of time. Prior to organ harvesting, the victim was infused with a pharmaceutical composition comprising a compound as described herein, resulting from the inventive method as described herein. Harvested organs for shipment were perfused with a perfusate containing a compound as described herein, and stored in a bath comprising a compound as described herein, resulting from the inventive method. Certain organs were continuously perfused with a pulsatile perfusion device, utilizing a perfusate containing a compound. Minimal deterioration of organ function occurred during the transport and upon implant and reperfusion of the organs *in situ.*

In another aspect, a surgical procedure to repair a heart valve required temporary cardioplegia and arterial occlusion. Prior to surgery, the patient was infused with 4 µg of a compound per kg body weight. Such treatment prevented hypoxic ischemic cellular damage, particularly after reperfusion.

In another aspect, in any surgical procedure, such as in cardiopulmonary bypass surgery, a compound as described herein, resulting from the inventive method can be used. In one embodiment, administration of a pharmaceutical composition comprising a compound as described herein, resulting from the inventive method as described above is performed prior to, during, and/or following the bypass procedure, to protect the function of brain, heart, and other organs.

In the foregoing examples in which a compound as described herein, resulting from the inventive method is used for *ex-vivo* applications, or to treat responsive cells such as neuronal tissue, retinal tissue, heart, lung, liver, kidney, small intestine, adrenal cortex, adrenal medulla, capillary endothelial, testes, ovary, or endometrial cells or tissue, provided is a pharmaceutical composition in dosage unit form adapted for protection or enhancement of responsive cells, tissues, or organs distal to the vasculature which comprises, per dosage unit, an effective non-toxic amount within the range from about 0.01 pg to 5 mg, 1 pg to 5 mg, 500pg to 5 mg, 1 ng to 5 mg, 500 ng to 5 mg, 1 µg to 5 mg, 500 µg to 5mg, or 1 mg to 5 mg of compound of the invention and a pharmaceutically acceptable carrier. In a preferred aspect, the amount of a compound as described herein, resulting from the inventive method is within the range from about 1 ng to 5mg.

In a further aspect, EPO administration was found to restore cognitive function in animals having undergone brain trauma. The compounds as described herein, resulting from the inventive method would be expected to have the same cellular protective effects as EPO. After a delay of either 5 days or 30 days, EPO was still able to restore function as compared to sham-treated animals, indicating the ability of a EPO to regenerate or restore brain activity. Thus, the description is also directed to the use of a compound as described herein, resulting from the inventive method for the preparation of a pharmaceutical composition for the treatment of brain trauma and other cognitive dysfunctions, including treatment well after the injury (*e*.*g*., three days, five days, a week, a month, or longer). The description is also directed to a method for the treatment of cognitive dysfunction following injury by administering an effective amount of a compound as described herein, resulting from the inventive method. Any compound as described herein, resulting from the inventive method as described herein may be used for this aspect.

Furthermore, this restorative aspect is directed to the use of any of the compounds as described herein, resulting from the inventive method herein for the preparation of a pharmaceutical composition for the restoration of cellular, tissue, or organ dysfunction, wherein treatment is initiated after, and well after, the initial insult responsible for the dysfunction. Moreover, treatment using a compound as described herein, resulting from the inventive method can span the course of the disease or condition during the acute phase as well as a chronic phase.

In the instance wherein a compound has erythropoietic activity, the compound may be administered systemically at a dosage between about 1, µg and about 100 µg /kg body weight, preferably about 5-50 µg/kg-body weight, most preferably about 10-30 µg/kg-body weight, per administration. This effective dose should be sufficient to achieve serum levels of the compound greater than about 10,000,15,000, or 20,000 mU/ml of serum after compound administration. Such serum levels may be achieved at about 1, 2, 3, 4, 5, 6, 7, 8, 9, or 10 hours post-administration. Such dosages may be repeated as necessary. For example, administration may be repeated daily, as long as clinically necessary, or after an appropriate interval, *e*.*g*., every 1 to 12 weeks, but preferably, every 1 to 3 weeks. The effective amount of compound and a pharmaceutically acceptable carrier may be packaged in a single dose vial or other container. The compound as described herein, resulting from the inventive method, however is nonerythropoietic, *i*.*e*., it is capable of exerting the activities described herein without causing an increase in hemoglobin concentration or hematocrit. Such a non-erythropoietic compound is especially preferred in instances wherein the methods are intended to be provided chronically. In another aspect, a compound as described herein, resulting from the inventive method is given at a dose greater than that of a corresponding dose (W/W) of natural erythropoietin which would be necessary to maximally stimulate erythropoiesis. As noted above, a compound as described herein, resulting from the inventive method not have erythropoietic activity, and therefore the above dosages expressed in units are merely exemplary for corresponding amounts of natural erythropoietin; herein above molar equivalents for dosages are provided which are applicable to any compound of the invention.

### EXAMPLES

The present invention may be better understood by reference to the following non-limiting examples, which are provided as exemplary of the invention. The following examples are presented in order to more fully illustrate the preferred embodiments of the invention. They should in no way be construed, however, as limiting the broad scope of the invention.

### Example 1

### Production of Carbamylated Erythropoietin

The starting material of the process in this example was purified recombinant human EPO.

First the protein concentration was adjusted by ultrafiltration for the purpose of keeping a low process volume. The protein concentration was adjusted to 3 mg/ml. The ultrafiltration was performed by means of a BioMax (Millipore) with a MWCO of 5 kDa.

After completion of the concentration step, the EPO solution was mixed with 0.5 M K-borate tetra hydrate 0.5 M K-cymate, at pH 9.0 the solution was incubated at 32°C for 24 hours.

The desalting of the reaction mixture of EPO and cyanate was performed by gelfiltration. The protein was desalted to a 25 mM Tris, 50 mM NaCl pH 8.5 buffer. A G-25 Fine (Amersham Biosciences) resin was employed.

Using a flow of 90 cm/h on an approximately 15 cm high column a sample load of approximately 20% of the column volume was applied.

The desalted carbamylated EPO was collected for further processing.
- At this point, the polymer/aggregate content was 7.3 %.

The next step was the removal of aggregates and polymers performed by a purification step using anion exchange. A SOURCE 30Q (Amersham Biosciences) resin was employed for the purification. Approximately 4.5 mg/ml of carbamylated EPO was applied to the column. The running buffer A was: 25 mM Tris, 50 mM NaCl pH 8.5, and elution buffer B: 25 mM Tris, 1 M NaCl pH 8.5. The gradient was performed with 0-30 % over 20 column volumes the main peak of carbamylated EPO was collected and pooled.

The pool from the purification step was adjusted to a concentration > 0.5 mg/ml and buffer changed to a 20 mM citrate, 100 mM NaCl buffer using a dia/ultrafiltration tangential flow filtration unit. The concentration and buffer change was performed on a 0.1m² BioMax (Millipore) with a MWCO of 5 kDa.

Finally the purified biopharmacutical drug substance was 0.22 µm filtrated using a Millipak filter (Millipore) to reduce germs.

The process resulted in carbamylated EPO with properties making it useful as a biopharmaceutical;
- The polymer/aggregate content was 0.5 % as determined by SEC-HPLC
- The carbamylated lysines was 100% as determined by aminoacid analysis
- The concentration was > 0.5 mg/ml

Characterization using MALDI-TOF for the determination of the change in the intact mass both of PNGase treated protein and for the protein with the N-glycans was performed. In addition MALDI-TOF peptide mass fingerprint analysis/LC-MS/MS analysis was performed as follows:
1. CEPO and EPO were purified on a POROS R1 column (POROS R1 reverse phase column material, PerSeptive Biosystems (1-1259-06)). The column material was stored in 50% HiPerSolv for HPLC VWR 152525R before use. The R1 column was equilibrated and washed in 5% formic acid (33015, Riedl de Haën). The samples were eluted from the column with Agilent MALDI HCCA quality matrix solution (G2037A). The intact mass was determined by analysis on a Bruker Reflex IV MALDI-TOF instrument.
2. 0.3 pmol CEPO and/or EPO were treated over night with 1 unit of PNGase F and PNGase F/O-glycosidase. Total mass was determined using MALDI-TOF
3. CEPO and EPO (1.5pmol) were reduced in solution with 50ul 10mM DTT, 50mM NH₄CO₃ and subsequently alkylated in 50ul 55 mM iodoacetamide, 50mM NH₄CO₃. The samples were purified on POROS R1 column before trypsin digestion. A fraction of the digested sample was purified on POROS R2 columns before MALDI-TOF analysis (POROS 50 R2 PerSeptive Biosystems (1-1159-05)). The R2 column was equilibrated and washed in 0.1% trifluoroacetic acid (99+% spectrometric grade, Aldrich 3 02031-100ml). The samples were eluted from the column with Agilent MALDI HCCA quality matrix solution (G2037A). The pool of peptides obtained by the tryptic digestion were treated with PNGase F and purified over POROS R2 columns, and characterized by MALDI-TOF.
4. CEPO and EPO were reduced in solution with DTT and alkylated with iodoacetamide. The samples were purified on POROS R1 column before Glu-C digestion. A fraction of the digested sample was purified on POROS R2 columns before MALDI-TOF analysis. The pool of peptides obtained by the Glu-C digestion were treated with PNGase F and purified over POROS R2 columns.
5. To rule out the possibility of having partly carbamylated CEPO, intact EPO and CEPO were digested with Lys-C. The samples were analysed using MALDI-TOF.

The conclusion was that the 8 lysines and the N-terminal were carbamylated. No other carbamylated amino acids were detected and no modifications of the glycans were detected.

### References:

### General protein identification by MS:

Mann M, Hojrup P, Roepstorff P. (1993) Use of mass spectrometric molecular weight information to identify proteins in sequence databases, Biol Mass Spectrom 22,338-345
Yates, J R, Speicher S, Griffin P R, Hunkapiller T. (1993) Peptide mass maps: a highly informative approach to protein identification, Anal Biochem 214, 397-408

### Intact mass:

Laugesen S, Roepstorff P. (2003) Combination of two matrices results in improved performance of MALDI MS for peptide mass mapping and protein analysis. J Am Soc Mass Spectrom. 14(9), 992-1002.

### Digest/grafit:

Larsen MR, Hojrup P, Roepstorff P. (2005) Characterization of gel-separated glycoproteins using two-step proteolytic digestion combined with sequential microcolumns and mass spectrometry. Mol Cell Proteomics. 4(2), 107-19

Additional studies were done to determine the degree and homogeneity of carbamylation of EPO, the specificity of carbamylation and identity of carbamylation sites, and the presence of potential unspecific modifications due to side reactions of the carbamylation and purification process. Samples were analyzed by total mass analysis of deglycosylated protein samples and by peptide mapping using endoproteases LysC and trypsin for digestion and LC/MS analysis for peptide evaluation.

### Example 2

### Total Mass Analysis

Analysis was performed on three EPO samples carbamylated using the method of Example 1. All three samples were purified following the carbamylation reaction using anion exchange, as described in example 1. One of the CEPO samples (designated CEPO-CMC) was prepared by CMC Biotech, from a 1 gram production scale (concentration: 0.82 mg/ml; buffer: 20 mM Na-citrate, 0.3 mM citric acid, 0.1 M NaCl, pH 6.9-7.3). The remaining two samples, designated CEPO-1 and CEPO-2, were prepared from a 70 mg laboratory production scale (concentration: 1.1 mg/ml; buffer: 25 mM Tris, 0.2.M NaCl, pH 8.3-8.7). These CEPO samples were compared to unmodified or starting EPO (concentration: 0.82 mg/ml; buffer: 2 mM Na-citrate, 0.3mM citric acid, 0.1 NaCl, pH6.9-7.3) and mock CEPO (EPO which has gone through the carbamylation process without the addition of K-cyanate) (concentration: 0.38 mg/ml; buffer: 20mM Na-citrate, 0.3 mM citric acid, 0.1 M NaCl, pH 6.9-7.3).

### ESI-mass spectrometry

Prior to the total mass analysis, the samples were enzymatically deglycosylated. Each sample was incubated overnight with 50 µg of N-glycosidase F (Prozyme from Glyko), recombinant neuraminidase from *A. ureafaciens* and O-glycosidase at a protein concentration of 0.5 mg/ml. Completeness of the deglycosylation reaction was checked by SDS-PAGE by loading 3 µg of each sample onto 12% Tris-glycine gels. The remaining material from each sample was used for mass analysis.

The deglycosylated samples were brought to a concentration of 4-5 M guanidinium hydrochloride by adding the appropriate volume of guanidinium hydrochloride stock solution and were subsequently desalted into a buffer containing 2% formic acid and 40% acenitril. The guanidinium hydrochloride was added in order to ensure high recovery of deglycosylated EPO and CEPO for mass spectrometric analysis. Mass measurement was performed with a Waters ESI-Q-Tof- or a Waters ESI-LCT-mass spectrometer provided with an ESI-nanospray source of ionization. Evaluation of data was conducted by automatic deconvolation and by manual evaluation of specific peaks of interest with Mass Lynx 4.0 software. Quantitation of the relative ratios of the differently carbamylated CEPO species was done by calculation of relative ratios based on the signal intensities recorded in the m/z spectrum.

The deglycosylation of the samples resulted in the N-linked sugars being removed completely. However, the release of the O-linked sugars was incomplete, especially for the CEPO samples.

As expected, due to the carbamylation, the CEPO samples showed different mass spectra as compared to the EPO and mock-CEPO samples. As shown in Table 2, deconvolution of the spectra resulted in masses for the major peaks as theoretically expected for the various samples. In all of the CEPO samples, only highly carbamylated CEPO molecules were found, with the major isoform being the fully carbamylated isoform with the complete carbamylation of the 8 lysines and the N-terminus, i. e, 9 carbamyl residues. The CEPO samples showed heterogeneity in containing additional isoforms corresponding to 8,10 and 11 carbamyl residues attached to the CEPO. The species containing 8 carbamyl residues would be designated as under-carbamylated, missing at least one carbamyl residue. The species with 10 and 11 carbamyl residues would be designated as over-carbamylated, where the extra carbamyl residues attached would be bound in a non-specific manner to an amino acid other than a lysine. There were some minor signals in the spectra of all of the samples but none were considered to be non-specifically modified species. Some of the minor signals were found in both the EPO and CEPO samples and were considered to be contaminants already present in the starting EPO.

**Table 2**

| Deconvolved Masses of Deglycosylated CEPO- and EPO- samples obtained in Total Mass Analysis | | | |
|---|---|---|---|
| **SAMPLE** | **Mass Theoretically Expected** | **Mass Obtained** | **Interpretation** |
| Starting EPO | 18239 | 18237 | Corresponds |
| Mock-CEPO | 18239 | 18239 | Corresponds |
| CEPO-CMC | 18626 (9x carb) | 18583 | 8x carb |
| | | **18626** | **9x carb** |
| | | 18669 | 10x carb |
| | | 18711 | 11x carb |
| | | 18991 | 9x carb + O-sugar |
| CEPO-1 | 18626 (9x carb) | 18583 | 8x carb |
| | | **18626** | **9x carb** |
| | | 18669 | 10x carb |
| | | 18711 | 11x carb |
| | | 18989 | 9x carb + O-sugar |
| CEPO-2 | 18626 (9x carb) | 18584 | 8x carb |
| | | **18626** | **9x carb** |
| | | 18669 | 10x carb |
| | | 18712 | 11x carb |
| | | 18992 | 9x carb + O-sugar |

Table 3 shows the relative ratios of the various isoforms. Under-carbamylated CEPO ranges from about 1.5 to 5.5% of the total CEPO depending on the sample and over-carbamylated CEPO ranges from about 11 to 22% depending on the sample. CEPO-1 and CEPO-2 have similar distribution of the isoforms while the CEPO-CMC has less under-carbamylated species as compared to the other two samples. Different production scales give rise to products with a different distribution, but as Table 3 shows for the laboratory production scale, the production may be repeated with similar outcome. As discussed earlier, at any given production scale, the distribution may be adjusted by adjusting the pooling from the anion exchange column.

The numbers in Table 3 represent the minimum ratio of under- and over-carbamylated CEPO. The reason for this is the degree of carbamylation (8-fold to 11- fold) may not specify the exact degree of under, full and over-carbamylated CEPO. For example, a CEPO molecule containing 8 carbamyl residues as determined by mass analysis may have only 7 carbamyl groups specifically linked to lysines and the remaining carbamyl residue would be non-specifically attached another amino acid. This situation would be considered only as under-carbamylated, even though there is non-specifically bound carbamyl groups. Conversely, a CEPO molecule containing 10 carbamyl residues may have attached to only 8 residues specifically and two are bound non-specifically. In this situation, the CEPO isoform is considered only over-carbamylated, even though all of the lysines are not carbamylated.

**Table 3**

| Degree and Heterogeneity of Carbamylation: Relative Ratios of Differently Carbamylated CEPO Species | | | | |
|---|---|---|---|---|
| **No.** | | **RELATIVE CONTENT** | | |
| | **Degree of Carbamylation** | **CEPO-CMC*** | **CEPO-1*** | **CEPO-2**** |
| 1 | 8x carb | 1.5% | 5.1% | 5.5% |
| 2 | **9x carb** | **77%** | **83.3%** | **83.6%** |
| 3 | 10x carb | 19.8% | 10.9% | 10.2% |
| 4 | 11x carb | 1.7% | 0.7% | 0.7% |
| 5 | E No. 2-4 (>9x) | 98.5% | 94.9% | 94.5% |
| 6 | Σ No. 3+4 (over carb.) | 21.5% | 11.6% | 10.9% |

| | | | | |
|---|---|---|---|---|
| * n=2 **n=,1 | | | | |

### Example 3

### LysC Peptide Mapping

Peptide map analysis of the EPO and CEPO samples was performed using endoproteases LysC and trypsin for fragmentation. All peptide map analyses were conducted with degycosylated peptides. The enzymatic deglycosylation of the peptides was performed simultaneously with the digestion with the endoprotease.

EPO and CEPO samples (about 150 µg each) were denatured and reduced by incubation with guanidinium hydrochloride and DTT. Free sulfhydryl groups were alkylated with iodoacetic acid. The alkylated samples were desalted and the buffer was exchanged to the appropriate buffer by using single use gel filtration columns.

The endoprotease, N-glycosidase and neuraminidase were added simultaneously to the alkylated EPO and CEPO samples. The samples were incubated overnight at 37°C. After incubation, about 5µg of each digest was applied to RP-HPLC/MS analysis using a Jupiter, C18 RP-column from Phenomenix coupled to an ESI-LCT from Waters. The UV signal at 220 nM and the total ion counts (TIC) in the mass spectrometer were recorded. For identification and quantification of the peptides obtained, the TIC was evaluated.

Because LysC would not be able to cleave carbamylated lysines, it would be expected that no fragments would be formed by digestion with LysC if all of the lysines are carbamylated, indicating specificity of carbamylation. In the case of under-carbamylation, specific fragments of CEPO should form. Table 4 lists the fragments theoretically formed from LysC digestion for EPO, fully and over-carbamylated CEPO and under-carbamylated CEPO.

**Table 4**

| LysC Peptide Mapping: Lists of Peptide/Fragments Theoretically Formed from Digestion of LysC of EPO, fully or over-carbamylated CEPO and under-carbamylated CEPO EPO | | | |
|---|---|---|---|
| **Peptide Name** | **Amino acids from - to** | **Mass** | **Amino acid Sequence** |
| K1 | 1-20 | 2399.3 | APPR LIDSR |
| | | | VLER YLLEAK |
| K2 | 21-45 | 2804.2 | EANITTGCAEHCS LNENEITVPDDTK |
| K3 | 46-52 | 926.5 | VNFYAWK |
| K4 | 53-97 | 5022.7 | RMEVGQQAVEV |
| | | | WQGLALLSEAVL |
| | | | RGQALLVNSSQP |
| | | | WEPLQLHVDK |
| K5 | 98-116 | 1954.2 | AVSGLR |
| | | | SLTTLLR |
| | | | ALGAQK |
| K6 (+ O-sugar) | 117-140 | 2863.3 | EAISPPDAASAAP |
| | | | LR TITADTFR K |
| K7 | 141-152 | 1498.8 | LFR VYSNFLR GK |
| K8 | 153-154 | 259.2 | LK |
| K9 | 155-165 | 1242.5 | LYTGEACRTGD |

**CEPO (fully carbamylated and over-carbamylated)**

| **Fragment Name** | **Amino acids from - to** | **Mass** |
|---|---|---|
| CEPO (9x carb) | 1-165 | 19227 |
| CEPO (10x carb) | 1-165 | 19270 |
| CEPO (11x carb) | 1-165 | 19313 |

**Under-carbamylated CEPO (8x carbamylation)**

| **Non-carbamylated amino group** | **Mass expected for N-terminal fragment** | **Mass expected for C-terminal fragment** |
|---|---|---|
| None (9x carbamylated) | 19227 | -- |
| N-terminus | 19184 | -- |
| Lys20 | 2443.9 | 16755.9 |
| Lys45 | 5274.9 | 13924.8 |
| Lys52 | 6227.0 | 12972.7 |
| Lys97 | 11276.8 | 7923.0 |
| Lys116 | 13257.1 | 5942.6 |
| Lys140 | 16147.2 | 3052.4 |
| Lys152 | 17672.0 | 1527.5 |
| Lys154 | 17956.4 | 1244.3 |

As expected the LysC peptide pattern obtained for EPO and CEPO samples digested with LysC were completely different. Digestion of starting EPO and mock-CEPO with LysC resulted in the peptide pattern as expected. In both samples, all of the peptides K1 to K9 could be identified. Peptide K5 and K1 were partially cleaved in an unspecific manner, in both the EPO and mock-CEPO. No significant additional peaks could be identified nor were peaks missing in the LysC map of mock-CEPO as compared to starting EPO. From these data, it can be concluded that no significant non-specific covalent modifications of the EPO protein occurred during the carbamylation and purification process.

The peptide maps of the CEPO samples had a different peptide pattern from the starting EPO. There was a single major peak and some minor peaks. As shown in Table 5, the masses obtained from the peaks correlated well with masses expected either for uncleaved CEPO or for fragments from LysC cleavage of under-carbamylated CEPO. The major peak (A) contained intact, deglycosylated, fully carbamylated CEPO (9x carbamyls) and over-carbamylated CEPO (10x carbamyls) (Table 5). The four minor peaks (B-E) contained under-carbamylated CEPO (8x carbamyls), with different peaks containing under-carbamylated CEPO not carbamylated at certain lysines. Peaks B and C contained under-carbamylated CEPO specifically lacking carbamylation at Lys45, while peaks D and E contained under-carbamylated CEPO specifically lacking carbamylation at Lys97 (Table 5).

**Table 5**

| LysC Peptide Mapping: Assignment of Masses Experimentally Obtained to CEPO Fragments theoretically deriving from Under-carbamylated CEPO | | | |
|---|---|---|---|
| **Peak No.** | **Mass obtained** | **Interpretation** | **Mass expected** |
| A | 19228 | CEPO (9x carb + O-sugar) | 19227 |
| | 19271 | CEPO (10x carb + O-sugar) | 19270 |
| B | 18867 | CEPO (9x carb w/o O-sugar) | 18862 |
| | 13926 | CEPO-fragment (aa46-165) | 13924.3 |
| C | 5276 | CEPO-fragment (aa1-45) | 5274.8 |
| D | 7924 | CEPO-fragment (aa98-165) | 7922.4 |
| E | 11279 | CEPO-fragment (aa1-97) | 11276.6 |

| | | | |
|---|---|---|---|
| There were some differences in the relative ratios of the peaks for the different CEPO samples. CEPO samples 1 and 2 had more prominent peaks D and E than CEPO-CMC, indicating that they contained more under-carbamylated CEPO species. | | | |

It was difficult to quantitate the minor peaks as related to the major peak due to technical limitations. However using the peak areas from UV-detection as a crude indication for the relative ratios of the various species, it could be estimated that under-carbamylated CEPO may account for less than 10% of the CEPO isoforms in the samples and that CEPO- and CEPO-2 have double the amount of under-carbamylated isoforms than the CEPO-CMC. Using the same procedure as was used for the total mass analysis, quantitation of the over-carbamylated species located in peak A was about 21% for CEPO-CMC and 12% for CEPO-1 and CEPO-2.

Overall, the data from the LysC peptide mapping is in good agreement with the total mass analysis described in Example 2. CEPO-CMC contained less under-carbamylated CEPO isoforms while CEPO-1 and CEPO-2 contained more over-carbamylated CEPO isoforms. This peptide mapping also indicated that lysine 45 and lysine 97 may represent sites of under-carbamylation.

### Example 4

### Trypsin Peptide Mapping

The digestion of the sample using trypsin is described in Example 3.

Digestion of EPO and mock CEPO with trypsin resulted in a peptide pattern as expected. In both samples, most of the peptides (T1 to T21) as expected for trypsin digestion could be identified, except for some small di- and tri-peptides (such as T21). See Table 6. As was the case with the LysC digestion, there were no significant additional peaks nor were peaks missing from the mock-CEPO, as compared to starting EPO. From these data, it was concluded that there were no non-specifically covalent modifications of the EPO protein during carbamylation and purification process.

The peptide patterns of the CEPO samples were different from the unmodified EPO. Trypsin normally cleaves at lysine and arginine, thus it would be expected that in the case of fully carbamylated EPO only fragmentation would occur by cleavage of the arginines. Thus, after the peptide pattern is obtained with trypsin, the specific carbamylation sites and unspecifically carbamylated peptides could be identified. The peptides expected from a tryptic digestion of CEPO molecules assuming cleavage at the arginines (R) only is set forth in Table 6.

**Table 6**

| List of Peptides Expected for EPO and CEPO digested with Trypsin EPO | | | |
|---|---|---|---|
| **Peptide name** | **Amino acid from - to** to | **Mass (MH)** | **Amino acid sequence** |
| T1 | 1-4 | 439.3 | APPR |
| T2 | 5-10 | 763.4 | LICDSR |
| T3 | 11-14 | 513.3 | VLER |
| T4 | 15-20 | 735.4 | YLLEAK |
| T5 | 21-45 | 2806.2 | EANITTGCAEHCS |
| | | | LNENITVPDDTK |
| T6 | 46-52 | 926.5 | VNFYAWK |
| T7 | 53-53 | 174.1 | R |
| T8 | 54-76 | 2525.3 | MEVGQQAVEVW |
| | | | QGLALLSEAVLR |
| T9 | 77-97 | 2359.2 | GQALLVNSSQPW |
| | | | EPLQLHVDK |
| T10 | 98-103 | 601.4 | AVSGLR |
| T11 | 104-110 | 802.5 | SLTTLLR |
| T11 | 111-116 | 586.3 | ALGAQK |
| T13 (+O-sugar) | 117-131 | 1829.8 | EAISPPDAASAAP |
| | | | LR |
| T14 | 132-139 | 923.5 | TITADTFR |
| T15 | 140-140 | 146.1 | K |
| T16 | 141-143 | 434.3 | LFR |
| T17 | 144-150 | 897.5 | VYSNFLR |
| T18 | 151-152 | 203.1 | GK |
| T19 | 153-154 | 259.2 | LK |
| T20 | 155-162 | 969.4 | LYTGEACR |
| T21 | 163-165 | 291.1 | TGD |

**CEPO (assumed cleavage only at Arg (R) due to complete carbamylation)**

| **Peptide Name** | **Amino acids from - to** | **Mass (MH)** | **Amino acid sequence** |
|---|---|---|---|
| R1 (1x carb) | 1-4 | 482.3 | APPR |
| R2 | 5-10 | 763.4 | LICDR |
| R3 | 11-14 | 515.3 | VLER |
| R4 (3x carb) | 15-53 | 4717.2 | YLLEAKEANITT |
| | | | GCAEHCSLNENIT |
| | | | VPDDTKVNFYA |
| | | | WKR |
| R5 | 54-76 | 2525.3 | MEVGQAVEVWQ |
| | | | GLALLSEAVLR |
| R6 (1x carb) | 77-103 | 2985.3 | GQALLVNSSQPW |
| | | | EPLQLHVDKAVS |
| | | | GLR |
| R7 | 104-110 | 802.5 | SLTTLLR |
| R8 (1x carb) (+ O- | 111-131 | 2441.1 | ALGAQKEAISPPD |
| sugar) | | | AASAAPLR |
| R9 | 132-139 | 923.5 | TITADTFR |
| R10 (1x carb) | 140-143 | 605.4 | KLFR |
| R11 | 144-150 | 897.5 | VYSNFLR |
| R12 (2x carb) | 151-162 | 1481.7 | GKLKLYTGEACR |
| R13 | 163-165 | 291.1 | TGD |

All of the peptides expected from trypsin cleavage were identified as major peaks in all of the CEPO samples, with the exception of the C-terminal peptide R13. This peptide was also not found in the starting EPO or mock-CEPO. The peaks of R1 to R12 resulting from specific cleavage of only arginines accounted for the vast majority of peptides detected in the CEPO samples. Furthermore, all lysine-containing peptides were detected almost exclusively in the fully carbamylated form. These data indicate a high degree of specific carbamylation at the lysines and N-terminal.

In addition to the major peptide, six minor peptides were also detected in all the CEPO samples. Three of the minor peptides were identified by mass analysis as a result of tryptic cleavage of over-carbamylated CEPO and the remaining three resulted from under-carbamylated CEPO.

Table 7 lists all the peptides identified in the tryptic maps of the three CEPO samples analyzed. The high abundant peptides, R1 to R12, formed from completely and specifically carbamylated EPO are in regular letters, the correctly carbamylated EPO is additionally denoted in bold type. Peptides most likely formed by cleavage of under-carbamylated CEPO are denoted in italic type and peptides formed by cleavage of over-carbamylated CEPO are denoted in underlined type.

**Table 7**

| Tryptic Peptide Map: List of Peptides Identified in Tryptic Peptide Map of CEPO | | | | |
|---|---|---|---|---|
| Peptide | Mass | Relative Ion Counts (%)* CEPO-1 | Relative Ion Counts (%)* CEPO-2 | Relative Ion Counts (%)* CEPO-CMC |
| **R1_1xcarb** | **482.25** | **1.28** | **1.09** | **1.15** |
| R2 | 763.35 | 7.87 | 8.49 | 8.15 |
| R3 | 515.31 | 2.78 | 2.58 | 2.66 |
| *R4_b1_1xcarb* *(T6+17_1xcarb* | *1125.6* | *0.17* | *0.2* | *0.15* |
| **R4_3xcarb** | **4717.2** | **1.5** | **0.80** | **1.32** |
| R5 | 2525.34 | 11.44 | 9.56 | 9.54 |
| R5_ox | 2541.34 | 0.15 | 0.15 | 0.21 |
| R5_Na | 2547.32 | 0.15 | 0.13 | 0.14 |
| R5_a1 | 1869.97 | 0.17 | 0.17 | 0.16 |
| R5_b1 | 673.38 | 0.29 | 0.27 | 0.25 |
| *T9=R6_a1* | *2359.24* | *0.62* | *0.54* | *0.26* |
| T10=R6_b1 | *602. 35* | *0. 95* | *0.8* | *0. 65* |
| **R6_1xcarb** | **2985.60** | **15.36** | **14.48** | **15.78** |
| R7 | 802.49 | 7.16 | 7.28 | 7.05 |
| R7 1 xcarb | 845.49 | +¹ | +¹ | +¹ |
| R8_1xcarb | 2076.10 | 0.16 | 0.15 | 0.16 |
| R8_1xcarb | 2076.10 | 0.5 | 0.44 | 0.63 |
| **R8-SAO-1xcar b** | **2441.1** | **9.19** | **9.34** | **9.20** |
| R9 | 923.47 | 9.85 | 11.11 | 10.58 |
| **R10_1xcarb** | **605.37** | **5.17** | **5.40** | **5.43** |
| R10 2xcarb | 648.37 | 0.02 | 0.03 | 0.03 |
| R11 | 897.47 | 9.82 | 10.70 | 10.30 |
| **R12_al_2xcar b** | **806.46** | **0.20** | **0.22** | **0.17** |
| ***R12_2xcarb*** | **1481.73** | **7.91** | **7.90** | **7.84** |
| R12 3xcarb | 1524.74 | 0.28 | 0.26 | 0.59 |
| R13 | 291.11 | -- | -- | -- |

| | | | | |
|---|---|---|---|---|
| *- intensity relative to the total number of counts in TIC 1- found by manual evaluation; signal confirmed as specific | | | | |

Referring to Table 7, the minor peaks T9 and T10 containing peptides R6_a1 and R6_b1, respectively, most likely derive from the cleavage of under-carbamylated CEPO molecules, which are not carbamylated at Lys97. Peptide R4_b1+1xcarb is formed if the Lys45 amino acid is not carbamylated. Peptide R6_a1 (peak T9) is more abundant in the CEPO-1 and CEPO-2 samples than the CEPO-CMC indicating that the former may have double the amount of under-carbamylated CEPO. Peptides R6_b1 and R4_b1+1xcarb were present in equal amounts in all CEPO samples.

Calculation of the relative content of under-carbamylated CEPO from these data was difficult due to technical limitations. However, taking all the observations together, it was estimated that the under-carbamylation species was about 10% as it was deduced from the total mass analysis and LysC peptide mapping.

Three other minor peptides which co-eluted with other peptides were interpreted by mass as containing one extra carbamyl residue, *i*.*e*., over-carbamylated CEPO. Peptides R10_2xcarb and R7_1xcarb were detected in trace amounts, where R12_3xcarb was found to have significant signal intensities. CEPO-CMC had a two-fold higher content of over-carbamylated CEPO species as CEPO-1 and CEPO-2. This is in agreement with the results from the total mass analysis. It could also be concluded from these data that the amino acid sequence of 151-62 of EPO is a site for unspecific carbamylation.

Again, for the same reasons as with the quantitation of under-carbamylation, the quantitation of over-carbamylated species was difficult. However, assuming that the ionization efficiency of the peptides only differing in degree of carbamylation is similar, it was calculated by the relative ion counts of the R12-derivatives that about 3-7% of the CEPO is over-carbamylated species. This is lower than the amount of over-carbamylated isoforms calculated by total mass analysis.

In general, the following can be concluded from the total mass analysis and peptide mapping data of the EPO and CEPO.

From the total mass analysis, the CEPO samples appeared to carbamylated to a rather high degree. About 95-98% of all molecules are fully carbamylated and contain at least 9 carbamyl residues (see Table 3). The LysC and tryptic mapping confirm the high degree of carbamylation at specific sites and that most likely over 95% of the CEPO molecules are fully carbamylated at the 8 lysines and the N-terminus.

The data also showed found four isoforms of CEPO. Species with 8, 9, 10 and 11 carbamyl residues were detected in the CEPO samples analyzed, with the 9 carbamyl isoform being the dominant species. A minor portion of the CEPO molecules contained 8 carbamyl residues instead of 9 and were considered under-carbamylated. For CEPO-1 and CEPO-2 these isoforms were about 5% of the total and for the CEPO-CMC, this isoform made up about 1.5% of the total CEPO molecules.

A more significant portion of the CEPO molecules were over-carbamylated, i. e., contain 10 or 11 carbamyl residues. Over -carbamylated CEPO ranges from about 11 % for EPO-1 and CEPO-2 to about 22% in CEPO-CMC.

The data from the peptide mapping showed a high degree of specificity of carbamylation at the 8 lysines and N-terminus. Moreover, the peptide mapping generally confirmed the results of the total mass analysis. At least about 90-95% of the CEPO molecules appeared to be specifically modified by carbamyl residues at all of the lysines and the N-terminus. However, this data also showed under- and over-carbamylation CEPO species. Due to some technical limitations, the exact ratio of under-carbamylated species was hard to determine, but it is estimated to be in the range of up to about 10% which is in agreement with the numbers found in the total mass analysis. Moreover, two distinct positions on the EPO, Lys45 and Lys97, were identified as the ones where lack of carbamylation was most likely to occur.

In both sets of peptide mapping, over-carbamylated species were also found. Again, due to technical limitations, the exact amount of over-carbamylated species was hard to quantitate. From the data obtained, it could be speculated that too little over-carbamylated species were detected in the peptide mapping. Only one-third to one-half of the amount of over-carbamylated species was identified by the peptide mapping as compared to the total mass analysis. A reasonable explanation for this discrepancy is that not all of the over-carbamylated peptides were identified at all or in the correct quantitiy. In the LysC map, an uncleaved CEPO species containing 10 carbamyl residues was detected in significant amounts and in the tryptic mapping three peptides were detected that contained one extra carbamyl residue. Two of these fragments were detected in only trace amounts. The third peptide, CEPO peptide amino acids 152-162, close to the C-terminus, appears to account for one-third of the over-carbamylation and may be a site for non-specific carbamylation in EPO.

No significant amounts of other non-specific (not carbamyl related) modifications were detected in the CEPO samples or in the mock CEPO samples by any analysis performed.

## Claims

1. A method for producing a carbamylated erythropoietin protein having less than about 40% aggregated protein and less than about 40% by weight of over- and under-carbamylated protein as measured by ESI-mass spectrometry, which method comprises contacting an amount of erythropoietin with an amount of cyanate at a temperature, pH, and for a time period sufficient for the amine groups on the lysines and the N-terminal amino acids of the erythropoietin to become at least about 90% carbamylated.

2. The method of claim 1, wherein the carbamylated erythropoietin protein is human erythropoietin.

3. The method of claim 1, wherein the carbamylated erythropoietin protein has less than about 30% aggregated protein.

4. The method of claim 1, wherein the carbamylated erythropoietin protein has less than about 20% aggregated protein.

5. The method of claim 1, wherein the carbamylated erythropoeitin protein has less than about 10% aggregated protein.

6. The method of claim 1, wherein the carbamylated erythropoietin protein has less than about 30% by weight of over- and under-carbamylated protein.

7. The method of claim 1, wherein the carbamylated erythropoietin protein has less than about 20% by weight of over- and under-carbamylated protein.

8. The method of claim 1, wherein the carbamylated erythropoietin protein has less than about 10% by weight of over- and under-carbamylated protein.

9. The method of claim 1, wherein the carbamylated erythropoietin protein has less than about 30% by weight of over-carbamylated protein.

10. The method of claim 1, wherein the carbamylated erythropoietin protein has less than about 20% by weight of over-carbamylated protein.

11. The method of claim 1, wherein the carbamylated erythropoietin protein has less than about 10% by weight of over-carbamylated protein.

12. The method of claim 1, wherein the concentration of erythropoietin protein contacted with the cyanate is from about 0.05 mg/ml to about 10 mg /ml.

13. The method of claim 1, wherein the concentration of erythropoietin protein contacted with the cyanate is about 2 mg/ml to about 5 mg/ml.

14. The method of claim 1, wherein the concentration of the cyanate is from about 0.05 M to about 10 M.

15. The method of claim 1, wherein the concentration of the cyanate is from about 0.05 M to about 2 M.

16. The method of claim 1, wherein the temperature ranges from about 0°C to about 60° C.

17. The method of claim 1, wherein the temperature ranges from about 30°C to about 34°C.

18. The method of claim 1, wherein the pH is from about 7 to about 11.

19. The method of claim 1, wherein the pH is from about 8 to about 10.

20. The method of claim 1, wherein the time period is from about 10 minutes to about 30 days.

21. The method of claim 1, wherein the time period is from about 1 hour to about 5 days.

22. The method of claim 1, wherein the erythropoietin protein is contacted with the cyanate in the presence of a buffer.

23. The method of claim 22, wherein the buffer is borate.

24. The method of claim 22, wherein the concentration of the buffer is from about 0.05 M to about 2 M.

25. The method of claim 22, wherein the concentration of the buffer is from about 0.1 M to about 1 M.

26. The method of claim 22, wherein the concentration of the buffer is about 0.5M.

27. The method of claim 1, wherein the concentration of the erythropoietin protein contacted with the cyanate is from about 0.05 mg/ml to about 10 mg/ml, the concentration of the cyanate is from about 0.05 M to about 10 M, the temperature ranges from about 0°C to about 60°C, the pH is from about 7 to about 11, and the time is from about 10 minutes to thirty days.

28. The method of claim 1, wherein the concentration of the erythropoietin protein contacted with the cyanate is from about 2 mg/ml to about 5 mg/ml, the concentration of the cyanate is from about 0.05 M to about 2 M, the temperature ranges from about 30°C to about 34°C, the pH is from about 8 to about 10, and the time is from about 1 hour to 5 days.

29. The method of claim 1, wherein the concentration of the erythropoietin protein contacted with the cyanate is about 3 mg/ml, the concentration of the cyanate is about 0.5 M, the temperature is about 32°C, the pH is about 9.0, and the time period is about 24 hours.

30. A method for producing a carbamylated erythropoietin protein having less than about 3% aggregated protein and less than about 40% by weight of over- and under-carbamylated protein as measured by ESI-mass spectrometry, comprising purifying the carbamylated erythropoietin using anion exchange, cation exchange, hydrophobic interaction chromatography, reverse phase chromatography, affinity chromatography or size exclusion chromatography.

31. The method of claim 30, wherein the carbamylated erythropoietin protein is human erythropoietin.

32. The method of claim 30, wherein at least about 90% of the amine residues on the lysines and the N-terminal amino acid of the erythropoietin are carbamylated.

33. The method of claim 30, wherein the carbamylated erythropoietin protein has less than about 2.5% aggregated protein.

34. The method of claim 3 0, wherein the carbamylated erythropoietin protein has about 0.5% or less aggregated protein.

35. The method of claim 30, wherein the carbamylated erythropoietin protein has less than about 30% by weight of over- and under-carbamylated protein.

36. The method of claim 30, wherein the carbamylated erythropoietin protein has less than about 20% by weight of over- and under-carbamylated protein.

37. The method of claim 30 wherein the carbamylated erythropoietin protein has less than about 10% by weight of over- and under-carbamylated protein.

38. The method of claim 30, wherein the carbamylated erythropoietin protein has less than about 30% by weight of over-carbamylated protein.

39. The method of claim 30, wherein the carbamylated erythropoietin protein has less than about 20% by weight of over-carbamylated protein.

40. The method of claim 30, wherein the carbamylated erythropoietin protein has less than about 10% by weight of over-carbamylated protein.

41. A method for producing a carbamylated erythropoietin protein having less than about 3% aggregated protein and less than about 40% by weight of over- and under-carbamylated protein as measured by ESI mass spectrometry, which method comprises:
(a) contacting an amount of an erythropoietin protein with an amount of cyanate at a temperature, pH, and for a time period sufficient for at least about 90% of the amine residues on the lysine and the N-terminal amino acids of the erythropoietin to become carbamylated; and
(b) purifying the carbamylated erythropoietin protein using anion exchange, cation exchange, hydrophobic interaction chromatography, reverse phase chromatography, affinity chromatography or size exclusion chromatography.

42. The method of claim 41, wherein the carbamylated erythropoietin protein is human erythropoietin.

43. The method of claim 41, wherein the carbamylated erythropoietin protein has less than about 2.5% aggregated protein.

44. The method of claim 41, wherein the carbamylated erythropoietin protein has about 0.5% or less aggregated protein.

45. The method of claim 41, wherein the carbamylated erythropoietin protein has less than about 30% by weight of over- and under-carbamylated protein.

46. The method of claim 41, wherein the carbamylated erythropoietin protein has less than about 20% by weight of over- and under-carbamylated protein.

47. The method of claim 41, wherein the carbarnylated erythropoietin protein has less than about 10% by weight of over- and under-carbamylated protein.

48. The method of claim 41, wherein the carbamylated erythropoietin protein has less than about 30% by weight of over-carbamylated protein.

49. The method of claim 41, wherein the carbamylated erythropoietin protein has less than about 20% by weight of over-carbamylated protein.

50. The method of claim 41, wherein the carbamylated erythropoietin protein has less than about 10% by weight of over-carbamylated protein.

51. The method of claim 41, wherein the concentration of erythropoietin protein contacted with the cyanate is from about 0.05 mg/ml to about 10 mg/ml.

52. The method of claim 41, wherein the concentration of erythropoietin protein contacted with the cyanate is from about 2 mg/ml to about 5 mg/ml.

53. The method of claim 41, wherein the concentration of erythropoietin protein contacted with the cyanate is about 3 mg/ml.

54. The method of claim 41, wherein the concentration of the cyanate is from about 0.05 M to about 10 M.

55. The method of claim 41, wherein the concentration of the cyanate is from about 0.05 M to about 2 M.

56. The method of claim 41, wherein the concentration of the cyanate is about 0.5 M.

57. The method of claim 41, wherein the temperature ranges from about 0°C to about 60° C.

58. The method of claim 41, wherein the temperature ranges from about 30°C to about 34°C.

59. The method of claim 41, wherein the temperature is about 32°C.

60. The method of claim 41, wherein the pH is from about 7 to about 11.

61. The method of claim 41, wherein the pH is from about 8 to about 10.

62. The method of claim 41, wherein the pH is about 9.

63. The method of claim 41, wherein the time period is from about 10 minutes to about 30 days.

64. The method of claim 41, wherein the time period is from about 1 hour to about 5 days.

65. The method of claim 41, wherein the time period is about 24 hours.

66. The method of claim 41, wherein the erythropoietin protein is contacted with the cyanate in the presence of a buffer.

67. The method of claim 66, wherein the buffer is borate.

68. The method of claim 66, wherein the concentration of the buffer is from about 0.05 M to about 2 M.

69. The method of claim 66, wherein the concentration of the buffer is from about 0.1 M to about 1 M.

70. The method of claim 66, wherein the concentration of the buffer is about 0.5M.

71. The method of claim 41, wherein the concentration of erythropoietin protein contacted with the cyanate is from about 0.05 mg/ml to about 10 mg/ml, the concentration of the cyanate is from about 0.05 M to about 10 M, the temperature ranges from about 0°C to about 60°C, the pH is from about 7 to about 11, and the time is from about 10 minutes to thirty days.

72. The method of claim 41, wherein the concentration of erythropoietin protein contacted with the cyanate is from about 2 mg/ml to about 5 mg/ml, the concentration of the cyanate is from about 0.05 M to about 2 M, the temperature ranges from about 30°C to about 34°C, the pH is from about 8 to about 10, and the time is from about 1 hour to 5 days.

73. The method of claim 34, wherein the concentration of the erythropoietin protein contacted with the cyanate is about 3 mg/ml, the concentration of the cyanate is about 0.5 M, the temperature is about 32°C, the pH is about 9.0, and the time period is about 24 hours.

74. A method for producing a carbamylated erythropoietin protein having less than about 40% aggregated protein and less than about 40% by weight of over- and under-carbamylated protein, which method comprises contacting an amount of erythropoietin with an amount of cyanate at a temperature, pH, and for a time period sufficient for the amine groups on the lysines and the N-terminal amino acids of the erythropoietin to become at least about 90% carbamylated and wherein the erythropoietin is contacted with the cyanate in the presence of potassium borate.

75. A method for producing a carbamylated erythropoietin protein having less than about 40% aggregated protein and less than about 40% by weight of over- and under-carbamylated protein, which method comprises contacting an amount of erythropoietin with an amount of cyanate at a temperature of 30°C to 34°C, pH, and for a time period sufficient for the amine groups on the lysines and the N-terminal amino acids of the erythropoietin to become at least about 90% carbamylated.

76. A method for producing a carbamylated erythropoietin protein having less than about 40% aggregated protein and less than about 40% by weight of over- and under-carbamylated protein, which method comprises contacting an amount of erythropoietin with an amount of cyanate at a temperature, pH, and for a time period sufficient for the amine groups on the lysines and the N-terminal amino acids of the erythropoietin to become at least about 90% carbamylated, and wherein the carbamylated erythropoietin protein is purified using anion exchange chromatography while maintaining the pH of both the running buffer and the elution buffer at 8.5 ± 0.2.

## Patentansprüche

1. Verfahren zur Herstellung eines carbamylierten Erythropoietinproteins mit weniger als etwa 40 % aggregiertem Protein und weniger als etwa 40 Gew.% über- und unter-carbamyliertem Protein, wie mittels ESI-Massenspektrometrie gemessen, wobei das Verfahren das In-Kontakt-Bringen einer Menge Erythropoietin mit einer Menge Cyanat bei einer Temperatur, einem pH und für einen Zeitraum umfasst, die ausreichend sind, damit die Amingruppen der Lysinreste und die N-terminalen Aminosäuren des Erythropoietins zu mindestens etwa 90 % carbamyliert werden.

2. Verfahren gemäss Anspruch 1, worin das carbamylierte Erythropoietinprotein menschliches Erythropoietin ist.

3. Verfahren gemäss Anspruch 1, worin das carbamylierte Erythropoietinprotein weniger als etwa 30 % aggregiertes Protein aufweist.

4. Verfahren gemäss Anspruch 1, worin das carbamylierte Erythropoietinprotein weniger als etwa 20 % aggregiertes Protein aufweist.

5. Verfahren gemäss Anspruch 1, worin das carbamylierte Erythropoietinprotein weniger als etwa 10 % aggregiertes Protein aufweist.

6. Verfahren gemäss Anspruch 1, worin das carbamylierte Erythropoietinprotein weniger als etwa 30 Gew.% über- und unter-carbamyliertes Protein aufweist.

7. Verfahren gemäss Anspruch 1, worin das carbamylierte Erythropoietinprotein weniger als etwa 20 Gew.% über- und unter-carbamyliertes Protein aufweist.

8. Verfahren gemäss Anspruch 1, worin das carbamylierte Erythropoietinprotein weniger als etwa 10 Gew.% über- und unter-carbamyliertes Protein aufweist.

9. Verfahren gemäss Anspruch 1, worin das carbamylierte Erythropoietinprotein weniger als etwa 30 Gew.% übercarbamyliertes Protein aufweist.

10. Verfahren gemäss Anspruch 1, worin das carbamylierte Erythropoietinprotein weniger als etwa 20 Gew.% übercarbamyliertes Protein aufweist.

11. Verfahren gemäss Anspruch 1, worin das carbamylierte Erythropoietinprotein weniger als etwa 10 Gew.% übercarbamyliertes Protein aufweist.

12. Verfahren gemäss Anspruch 1, worin die Konzentration des Erythropoietinproteins, das mit dem Cyanat in Kontakt gebracht wird, von etwa 0,05 bis etwa 10 mg/ml beträgt.

13. Verfahren gemäss Anspruch 1, worin die Konzentration des Erythropoietinproteins, das mit dem Cyanat in Kontakt gebracht wird, von etwa 2 bis etwa 5 mg/ml beträgt.

14. Verfahren gemäss Anspruch 1, worin die Konzentration des Cyanats von etwa 0,05 bis etwa 10 M beträgt.

15. Verfahren gemäss Anspruch 1, worin die Konzentration des Cyanats von etwa 0,05 bis etwa 2 M beträgt.

16. Verfahren gemäss Anspruch 1, worin die Temperatur von etwa 0 bis etwa 60°C reicht.

17. Verfahren gemäss Anspruch 1, worin die Temperatur von etwa 30 bis etwa 34°C reicht.

18. Verfahren gemäss Anspruch 1, worin der pH von etwa 7 bis etwa 11 beträgt.

19. Verfahren gemäss Anspruch 1, worin der pH von etwa 8 bis etwa 10 beträgt.

20. Verfahren gemäss Anspruch 1, worin der Zeitraum von etwa 10 Minuten bis etwa 30 Tage beträgt.

21. Verfahren gemäss Anspruch 1, worin der Zeitraum von etwa 1 Stunde bis etwa 5 Tage beträgt.

22. Verfahren gemäss Anspruch 1, worin das Erythropoietinprotein in Gegenwart eines Puffers mit dem Cyanat in Kontakt gebracht wird.

23. Verfahren gemäss Anspruch 22, worin der Puffer Borat ist.

24. Verfahren gemäss Anspruch 22, worin die Konzentration des Puffers von etwa 0,05 bis etwa 2 M beträgt.

25. Verfahren gemäss Anspruch 22, worin die Konzentration des Puffers von etwa 0,1 bis etwa 1 M beträgt.

26. Verfahren gemäss Anspruch 22, worin die Konzentration des Puffers etwa 0,5 M beträgt.

27. Verfahren gemäss Anspruch 1, worin die Konzentration des Erythropoietinproteins, das mit dem Cyanat in Kontakt gebracht wird, von etwa 0,05 bis etwa 10 mg/ml beträgt, die Konzentration des Cyanats von etwa 0,05 bis etwa 10 M beträgt, die Temperatur von etwa 0 bis etwa 60°C reicht, der pH von etwa 7 bis etwa 11 beträgt und die Zeit von etwa 10 Minuten bis 30 Tage beträgt.

28. Verfahren gemäss Anspruch 1, worin die Konzentration des Erythropoietinproteins, das mit dem Cyanat in Kontakt gebracht wird, von etwa 2 bis etwa 5 mg/ml beträgt, die Konzentration des Cyanats von etwa 0,05 bis etwa 2 M beträgt, die Temperatur von etwa 30 bis etwa 34°C reicht, der pH von etwa 8 bis etwa 10 beträgt und die Zeit von etwa 1 Stunde bis 5 Tage beträgt.

29. Verfahren gemäss Anspruch 1, worin die Konzentration des Erythropoietinproteins, das mit dem Cyanat in Kontakt gebracht wird, etwa 3 mg/ml beträgt, die Konzentration des Cyanats etwa 0,5 M beträgt, die Temperatur etwa 32°C beträgt, der pH etwa 9,0 beträgt und der Zeitraum etwa 24 Stunden beträgt.

30. Verfahren zur Herstellung eines carbamylierten Erythropoietinproteins mit weniger als etwa 3 % aggregiertem Protein und weniger als etwa 40 Gew.% über- und unter-carbamyliertem Protein, wie mittels ESI-Massenspektrometrie gemessen, umfassend die Aufreinigung des carbamylierten Erythropoietins unter Verwendung von Anionenaustausch, Kationenaustausch, hydrophober Wechselwirkungschromatografie, Umkehrphasenchromatografie, Affinitätschromatografie oder Grössenausschlusschromatografie.

31. Verfahren gemäss Anspruch 30, worin das carbamylierte Erythropoietinprotein menschliches Erythropoietin ist.

32. Verfahren gemäss Anspruch 30, worin mindestens etwa 90 % der Aminreste der Lysinreste und der N-terminalen Aminosäure des Erythropoietins carbamyliert sind.

33. Verfahren gemäss Anspruch 30, worin das carbamylierte Erythropoietinprotein weniger als etwa 2,5 % aggregiertes Protein aufweist.

34. Verfahren gemäss Anspruch 30, worin das carbamylierte Erythropoietinprotein etwa 0,5 % oder weniger aggregiertes Protein aufweist.

35. Verfahren gemäss Anspruch 30, worin das carbamylierte Erythropoietinprotein weniger als etwa 30 Gew.% über- und unter-carbamyliertes Protein aufweist.

36. Verfahren gemäss Anspruch 30, worin das carbamylierte Erythropoietinprotein weniger als etwa 20 Gew.% über- und unter-carbamyliertes Protein aufweist.

37. Verfahren gemäss Anspruch 30, worin das carbamylierte Erythropoietinprotein weniger als etwa 10 Gew.% über- und unter-carbamyliertes Protein aufweist.

38. Verfahren gemäss Anspruch 30, worin das carbamylierte Erythropoietinprotein weniger als etwa 30 Gew.% übercarbamyliertes Protein aufweist.

39. Verfahren gemäss Anspruch 30, worin das carbamylierte Erythropoietinprotein weniger als etwa 20 Gew.% übercarbamyliertes Protein aufweist.

40. Verfahren gemäss Anspruch 30, worin das carbamylierte Erythropoietinprotein weniger als etwa 10 Gew.% übercarbamyliertes Protein aufweist.

41. Verfahren zur Herstellung eines carbamylierten Erythropoietinproteins mit weniger als etwa 3 % aggregiertem Protein und weniger als etwa 40 Gew.% über- und unter-carbamyliertem Protein, wie mittels ESI-Massenspektrometrie gemessen, wobei das Verfahren umfasst:
(a) In-Kontakt-Bringen einer Menge eines Erythropoietinproteins mit einer Menge Cyanat bei einer Temperatur, einem pH und für einen Zeitraum, die ausreichend sind, damit mindestens etwa 90 % der Aminreste der Lysinreste und der N-terminalen Aminosäuren des Erythropoietins carbamyliert werden; und
(b) Aufreinigen des carbamylierten Erythropoietinproteins unter Verwendung von Anionenaustausch, Kationenaustausch, hydrophober Wechselwirkungschromatografie, Umkehrphasenchromatografie, Affinitätschromatografie oder Grössenausschlusschromatografie.

42. Verfahren gemäss Anspruch 41, worin das carbamylierte Erythropoietinprotein menschliches Erythropoietin ist.

43. Verfahren gemäss Anspruch 41, worin das carbamylierte Erythropoietinprotein weniger als etwa 2,5 % aggregiertes Protein aufweist.

44. Verfahren gemäss Anspruch 41, worin das carbamylierte Erythropoietinprotein etwa 0,5 % oder weniger aggregiertes Protein aufweist.

45. Verfahren gemäss Anspruch 41, worin das carbamylierte Erythropoietinprotein weniger als etwa 30 Gew.% über- und unter-carbamyliertes Protein aufweist.

46. Verfahren gemäss Anspruch 41, worin das carbamylierte Erythropoietinprotein weniger als etwa 20 Gew.% über- und unter-carbamyliertes Protein aufweist.

47. Verfahren gemäss Anspruch 41, worin das carbamylierte Erythropoietinprotein weniger als etwa 10 Gew.% über- und unter-carbamyliertes Protein aufweist.

48. Verfahren gemäss Anspruch 41, worin das carbamylierte Erythropoietinprotein weniger als etwa 30 Gew.% übercarbamyliertes Protein aufweist.

49. Verfahren gemäss Anspruch 41, worin das carbamylierte Erythropoietinprotein weniger als etwa 20 Gew.% übercarbamyliertes Protein aufweist.

50. Verfahren gemäss Anspruch 41, worin das carbamylierte Erythropoietinprotein weniger als etwa 10 Gew.% übercarbamyliertes Protein aufweist.

51. Verfahren gemäss Anspruch 41, worin die Konzentration des Erythropoietinproteins, das mit dem Cyanat in Kontakt gebracht wird, von etwa 0,05 bis etwa 10 mg/ml beträgt.

52. Verfahren gemäss Anspruch 41, worin die Konzentration des Erythropoietinproteins, das mit dem Cyanat in Kontakt gebracht wird, von etwa 2 bis etwa 5 mg/ml beträgt.

53. Verfahren gemäss Anspruch 41, worin die Konzentration des Erythropoietinproteins, das mit dem Cyanat in Kontakt gebracht wird, etwa 3 mg/ml beträgt.

54. Verfahren gemäss Anspruch 41, worin die Konzentration des Cyanats von etwa 0,05 bis etwa 10 M beträgt.

55. Verfahren gemäss Anspruch 41, worin die Konzentration des Cyanats von etwa 0,05 bis etwa 2 M beträgt.

56. Verfahren gemäss Anspruch 41, worin die Konzentration des Cyanats etwa 0,5 M beträgt.

57. Verfahren gemäss Anspruch 41, worin die Temperatur von etwa 0 bis etwa 60°C reicht.

58. Verfahren gemäss Anspruch 41, worin die Temperatur von etwa 30 bis etwa 34°C reicht.

59. Verfahren gemäss Anspruch 41, worin die Temperatur etwa 32°C beträgt.

60. Verfahren gemäss Anspruch 41, worin der pH von etwa 7 bis etwa 11 beträgt.

61. Verfahren gemäss Anspruch 41, worin der pH von etwa 8 bis etwa 10 beträgt.

62. Verfahren gemäss Anspruch 41, worin der pH etwa 9 beträgt.

63. Verfahren gemäss Anspruch 41, worin der Zeitraum von etwa 10 Minuten bis etwa 30 Tage beträgt.

64. Verfahren gemäss Anspruch 41, worin der Zeitraum von etwa 1 Stunde bis etwa 5 Tage beträgt.

65. Verfahren gemäss Anspruch 41, worin der Zeitraum etwa 24 Stunden beträgt.

66. Verfahren gemäss Anspruch 41, worin das Erythropoietinprotein mit dem Cyanat in Gegenwart eines Puffers in Kontakt gebracht wird.

67. Verfahren gemäss Anspruch 66, worin der Puffer Borat ist.

68. Verfahren gemäss Anspruch 66, worin die Konzentration des Puffers von etwa 0,05 bis etwa 2 M beträgt.

69. Verfahren gemäss Anspruch 66, worin die Konzentration des Puffers von etwa 0,1 bis etwa 1 M beträgt.

70. Verfahren gemäss Anspruch 66, worin die Konzentration des Puffers etwa 0,5 M beträgt.

71. Verfahren gemäss Anspruch 41, worin die Konzentration des Erythropoietinproteins, das mit dem Cyanat in Kontakt gebracht wird, von etwa 0,05 bis etwa 10 mg/ml beträgt, die Konzentration des Cyanats von etwa 0,05 bis etwa 10 M beträgt, die Temperatur von etwa 0 bis etwa 60°C reicht, der pH von etwa 7 bis etwa 11 beträgt und die Zeit von etwa 10 Minuten bis 30 Tage beträgt.

72. Verfahren gemäss Anspruch 41, worin die Konzentration des Erythropoietinproteins, das mit dem Cyanat in Kontakt gebracht wird, von etwa 2 bis etwa 5 mg/ml beträgt, die Konzentration des Cyanats von etwa 0,05 bis etwa 2 M beträgt, die Temperatur von etwa 30 bis etwa 34°C reicht, der pH von etwa 8 bis etwa 10 beträgt und die Zeit von etwa 1 Stunde bis 5 Tage beträgt.

73. Verfahren gemäss Anspruch 34 worin die Konzentration des Erythropoietinproteins, das mit dem Cyanat in Kontakt gebracht wird, etwa 3 mg/ml beträgt, die Konzentration des Cyanats etwa 0,5 M beträgt, die Temperatur etwa 32°C beträgt, der pH etwa 9,0 beträgt und der Zeitraum etwa 24 Stunden beträgt.

74. Verfahren zur Herstellung eines carbamylierten Erythropoietinproteins mit weniger als etwa 40 % aggregiertem Protein und weniger als etwa 40 Gew.% über- und unter-carbamyliertem Protein, wobei das Verfahren das In-Kontakt-Bringen einer Menge Erythropoietin mit einer Menge Cyanat bei einer Temperatur, einem pH und für einen Zeitraum umfasst, die ausreichend sind, damit die Amingruppen der Lysinreste und die N-terminalen Aminosäuren des Erythropoietins zu mindestens etwa 90 % carbamyliert werden, und worin das Erythropoietin mit dem Cyanat in Gegenwart von Kaliumborat in Kontakt gebracht wird.

75. Verfahren zur Herstellung eines carbamylierten Erythropoietinproteins mit weniger als etwa 40 % aggregiertem Protein und weniger als etwa 40 Gew.% über- und unter-carbamyliertem Protein, wobei das Verfahren das In-Kontakt-Bringen einer Menge Erythropoietin mit einer Menge Cyanat bei einer Temperatur von 30 bis 34°C, einem pH und für einen Zeitraum umfasst, die ausreichend sind, damit die Amingruppen der Lysinreste und die N-terminalen Aminosäuren des Erythropoietins zu mindestens etwa 90 % carbamyliert werden.

76. Verfahren zur Herstellung eines carbamylierten Erythropoietinproteins mit weniger als etwa 40 % aggregiertem Protein und weniger als etwa 40 Gew.% über- und unter-carbamyliertem Protein, wobei das Verfahren das In-Kontakt-Bringen einer Menge Erythropoietin mit einer Menge Cyanat bei einer Temperatur, einem pH und für einen Zeitraum umfasst, die ausreichend sind, damit die Amingruppen der Lysinreste und die N-terminalen Aminosäuren des Erythropoietins zu mindestens etwa 90 % carbamyliert werden, und worin das carbamylierte Erythropoietinprotein unter Verwendung von Anionenaustauschchromatografie gereinigt wird, während der pH sowohl des Laufpuffers als auch des Elutionspuffers auf 8,5 ± 0,2 gehalten wird.

## Revendications

1. Procédé de production d'une protéine érythropoïétine carbamylée ayant moins d'environ 40 % de protéine agrégée et moins d'environ 40 % en masse de protéine sur- et sous-carbamylée selon une mesure par spectrométrie de masse ESI, ce procédé comprenant la mise en contact d'une quantité d'érythropoïétine avec une quantité de cyanate à une température, un pH et pendant un intervalle de temps suffisants pour que les groupes amine des lysines et des aminoacides N-terminaux de l'érythropoïétine se carbamylent à au moins environ 90 %.

2. Procédé selon la revendication 1, dans lequel la protéine érythropoïétine carbamylée est une érythropoïétine humaine;

3. Procédé selon la revendication 1, dans lequel la protéine érythropoïétine carbamylée a moins d'environ 30 % de protéine agrégée.

4. Procédé selon la revendication 1, dans lequel la protéine érythropoïétine carbamylée a moins d'environ 20 % de protéine agrégée.

5. Procédé selon la revendication 1, dans lequel la protéine érythropoïétine carbamylée a moins d'environ 10 % de protéine agrégée.

6. Procédé selon la revendication 1, dans lequel la protéine érythropoïétine carbamylée a moins d'environ 30 % en masse de protéine sur- et sous-carbamylée.

7. Procédé selon la revendication 1, dans lequel la protéine érythropoïétine carbamylée a moins d'environ 20 % en masse de protéine sur- et sous-carbamylée.

8. Procédé selon la revendication 1, dans lequel la protéine érythropoïétine carbamylée a moins d'environ 10 % en masse de protéine sur- et sous-carbamylée.

9. Procédé selon la revendication 1, dans lequel la protéine érythropoïétine carbamylée a moins d'environ 30 % en masse de protéine sur-carbamylée.

10. Procédé selon la revendication 1, dans lequel la protéine érythropoïétine carbamylée a moins d'environ 20 % en masse de protéine sur-carbamylée.

11. Procédé selon la revendication 1, dans lequel la protéine érythropoïétine carbamylée a moins d'environ 10 % en masse de protéine sur-carbamylée.

12. Procédé selon la revendication 1, dans lequel la concentration de la protéine érythropoïétine mise en contact avec le cyanate est d'environ 0,05 mg/ml à environ 10 mg/ml.

13. Procédé selon la revendication 1, dans lequel la concentration de la protéine érythropoïétine mise en contact avec le cyanate est d'environ 2 mg/ml à environ 5 mg/ml.

14. Procédé selon la revendication 1, dans lequel la concentration du cyanate est d'environ 0,05 M à environ 10 M.

15. Procédé selon la revendication 1, dans lequel la concentration du cyanate est d'environ 0,05 M à environ 2 M.

16. Procédé selon la revendication 1, dans lequel la température se situe dans un domaine d'environ 0°C à environ 60°C.

17. Procédé selon la revendication 1, dans lequel la température se situe dans un domaine d'environ 30°C à environ 34°C.

18. Procédé selon la revendication 1, dans lequel le pH est d'environ 7 à environ 11.

19. Procédé selon la revendication 1, dans lequel le pH est d'environ 8 à environ 10.

20. Procédé selon la revendication 1, dans lequel l'intervalle de temps est d'environ 10 minutes à environ 30 jours.

21. Procédé selon la revendication 1, dans lequel l'intervalle de temps est d'environ 1 heure à environ 5 jours.

22. Procédé selon la revendication 1, dans lequel la protéine érythropoïétine est mise en contact avec le cyanate en présence d'un tampon.

23. Procédé selon la revendication 22, dans lequel le tampon est un borate.

24. Procédé selon la revendication 22, dans lequel la concentration du tampon est d'environ 0,05 M à environ 2 M.

25. Procédé selon la revendication 22, dans lequel la concentration du tampon est d'environ 0,1 M à environ 1 M.

26. Procédé selon la revendication 22, dans lequel la concentration du tampon est d'environ 0,5 M.

27. Procédé selon la revendication 1, dans lequel la concentration de la protéine érythropoïétine mise en contact avec le cyanate est d'environ 0,05 mg/ml à environ 10 mg/ml, la concentration du cyanate est d'environ 0,05 M à environ 10 M, la température se situe dans un domaine d'environ 0°C à environ 60°C, le pH est d'environ 7 à environ 11, et le temps est d'environ 10 minutes à environ trente jours.

28. Procédé selon la revendication 1, dans lequel la concentration de la protéine érythropoïétine mise en contact avec le cyanate est d'environ 2 mg/ml à environ 5 mg/ml, la concentration du cyanate est d'environ 0,05 M à environ 2 M, la température se situe dans un domaine d'environ 30°C à environ 34°C, le pH est d'environ 8 à environ 10, et le temps est d'environ 1 heure à environ 5 jours.

29. Procédé selon la revendication 1, dans lequel la concentration de la protéine érythropoïétine mise en contact avec le cyanate est d'environ 3 mg/ml, la concentration du cyanate est d'environ 0,5 M, la température est d'environ 32°C, le pH est d'environ 9,0, et l'intervalle de temps est d'environ 24 heures.

30. Procédé de production d'une protéine érythropoïétine carbamylée ayant moins d'environ 3 % de protéine agrégée et moins d'environ 40 % en masse de protéine sur- et sous-carbamylée selon une mesure par spectrométrie de masse ESI, comprenant la purification de l'érythropoïétine carbamylée utilisant un échange d'anions, un échange de cations, une chromatographie par interaction hydrophobe, une chromatographie en phase inverse, une chromatographie d'affinité ou une chromatographie d'exclusion stérique.

31. Procédé selon la revendication 30, dans lequel la protéine érythropoïétine carbamylée est une érythropoïétine humaine.

32. Procédé selon la revendication 30, dans lequel au moins environ 90 % des résidus amine des lysines et de l'aminoacide N-terminal de l'érythropoïétine sont carbamylés.

33. Procédé selon la revendication 30, dans lequel la protéine érythropoïétine carbamylée a moins d'environ 2,5 % de protéine agrégée.

34. Procédé selon la revendication 30, dans lequel la protéine érythropoïétine carbamylée a environ 0,5 % ou moins de protéine agrégée.

35. Procédé selon la revendication 30, dans lequel la protéine érythropoïétine carbamylée a moins d'environ 30 % en masse de protéine sur- et sous-carbamylée.

36. Procédé selon la revendication 30, dans lequel la protéine érythropoïétine carbamylée a moins d'environ 20 % en masse de protéine sur- et sous-carbamylée.

37. Procédé selon la revendication 30, dans lequel la protéine érythropoïétine carbamylée a moins d'environ 10 % en masse de protéine sur- et sous-carbamylée.

38. Procédé selon la revendication 30, dans lequel la protéine érythropoïétine carbamylée a moins d'environ 30 % en masse de protéine sur-carbamylée.

39. Procédé selon la revendication 30, dans lequel la protéine érythropoïétine carbamylée a moins d'environ 20 % en masse de protéine sur-carbamylée.

40. Procédé selon la revendication 30, dans lequel la protéine érythropoïétine carbamylée a moins d'environ 10 % en masse de protéine sur-carbamylée.

41. Procédé de production d'une quantité de protéine érythropoïétine carbamylée ayant moins d'environ 3 % de protéine agrégée et moins d'environ 40 % en masse de protéine sur- et sous-carbamylée selon une mesure par spectrométrie de masse ESI, ce procédé comprenant:
(a) la mise en contact d'une protéine érythropoïétine avec une quantité de cyanate à une température, un pH et pendant un intervalle de temps suffisants pour qu'au moins environ 90 % des groupes amine de la lysine et des aminoacides N-terminaux de l'érythropoïétine se carbamylent; et
(b) la purification de la protéine érythropoïétine carbamylée utilisant un échange d'anions, un échange de cations, une chromatographie par interaction hydrophobe, une chromatographie en phase inverse, une chromatographie d'affinité ou une chromatographie d'exclusion stérique.

42. Procédé selon la revendication 41, dans lequel la protéine érythropoïétine carbamylée est une érythropoïétine humaine.

43. Procédé selon la revendication 41, dans lequel la protéine érythropoïétine carbamylée a moins d'environ 2,5 % de protéine agrégée.

44. Procédé selon la revendication 41, dans lequel la protéine érythropoïétine carbamylée a environ 0,5 % ou moins de protéine agrégée.

45. Procédé selon la revendication 41, dans lequel la protéine érythropoïétine carbamylée a moins d'environ 30 % en masse de protéine sur- et sous-carbamylée.

46. Procédé selon la revendication 41, dans lequel la protéine érythropoïétine carbamylée a moins d'environ 20 % en masse de protéine sur- et sous-carbamylée.

47. Procédé selon la revendication 41, dans lequel la protéine érythropoïétine carbamylée a moins d'environ 10 % en masse de protéine sur- et sous-carbamylée.

48. Procédé selon la revendication 41, dans lequel la protéine érythropoïétine carbamylée a moins d'environ 30 % en masse de protéine sur-carbamylée.

49. Procédé selon la revendication 41, dans lequel la protéine érythropoïétine carbamylée a moins d'environ 20 % en masse de protéine sur-carbamylée.

50. Procédé selon la revendication 41, dans lequel la protéine érythropoïétine carbamylée a moins d'environ 10 % en masse de protéine sur-carbamylée.

51. Procédé selon la revendication 41, dans lequel la concentration de la protéine érythropoïétine mise en contact avec le cyanate est d'environ 0,05 mg/ml à environ 10 mg/ml.

52. Procédé selon la revendication 41, dans lequel la concentration de la protéine érythropoïétine mise en contact avec le cyanate est d'environ 2 mg/ml à environ 5 mg/ml.

53. Procédé selon la revendication 41, dans lequel la concentration de la protéine érythropoïétine mise en contact avec le cyanate est d'environ 3 mg/ml.

54. Procédé selon la revendication 41, dans lequel la concentration du cyanate est d'environ 0,05 M à environ 10 M.

55. Procédé selon la revendication 41, dans lequel la concentration du cyanate est d'environ 0,05 M à environ 2 M.

56. Procédé selon la revendication 41, dans lequel la concentration du cyanate est d'environ 0,5 M.

57. Procédé selon la revendication 41, dans lequel la température se situe dans un domaine d'environ 0°C à environ 60°C.

58. Procédé selon la revendication 41, dans lequel la température se situe dans un domaine d'environ 30°C à environ 34°C.

59. Procédé selon la revendication 41, dans lequel la température est d'environ 32°C.

60. Procédé selon la revendication 41, dans lequel le pH est d'environ 7 à environ 11.

61. Procédé selon la revendication 41, dans lequel le pH est d'environ 8 à environ 10.

62. Procédé selon la revendication 41, dans lequel le pH est d'environ 9.

63. Procédé selon la revendication 41, dans lequel l'intervalle de temps est d'environ 10 minutes à environ 30 jours.

64. Procédé selon la revendication 41, dans lequel l'intervalle de temps est d'environ 1 heure à environ 5 jours.

65. Procédé selon la revendication 41, dans lequel l'intervalle de temps est d'environ 24 heures.

66. Procédé selon la revendication 41, dans lequel la protéine érythropoïétine est mise en contact avec le cyanate en présence d'un tampon.

67. Procédé selon la revendication 66, dans lequel le tampon est un borate.

68. Procédé selon la revendication 66, dans lequel la concentration du tampon est d'environ 0,05 M à environ 2 M.

69. Procédé selon la revendication 66, dans lequel la concentration du tampon est d'environ 0,1 M à environ 1 M.

70. Procédé selon la revendication 66, dans lequel la concentration du tampon est d'environ 0,5 M.

71. Procédé selon la revendication 41, dans lequel la concentration de la protéine érythropoïétine mise en contact avec le cyanate est d'environ 0,05 mg/ml à environ 10 mg/ml, la concentration du cyanate est d'environ 0,05 M à environ 10 M, la température se situe dans un domaine d'environ 0°C à environ 60°C, le pH est d'environ 7 à environ 11, et le temps est d'environ 10 minutes à environ trente jours.

72. Procédé selon la revendication 41, dans lequel la concentration de la protéine érythropoïétine mise en contact avec le cyanate est d'environ 2 mg/ml à environ 5 mg/ml, la concentration du cyanate est d'environ 0,05 M à environ 2 M, la température se situe dans un domaine d'environ 30°C à environ 34°C, le pH est d'environ 8 à environ 10, et le temps est d'environ 1 heure à environ 5 jours.

73. Procédé selon la revendication 34, dans lequel la concentration de la protéine érythropoïétine mise en contact avec le cyanate est d'environ 3 mg/ml, la concentration du cyanate est d'environ 0,5 M, la température est d'environ 32°C, le pH est d'environ 9,0, et l'intervalle de temps est d'environ 24 heures.

74. Procédé de production d'une protéine érythropoïétine carbamylée ayant moins d'environ 40 % de protéine agrégée et moins d'environ 40 % en masse de protéine sur- et sous-carbamylée, ce procédé comprenant la mise en contact d'une quantité d'érythropoïétine avec une quantité de cyanate à une température, un pH et pendant un intervalle de temps suffisants pour que les groupes amine des lysines et des aminoacides N-terminaux de l'érythropoïétine se carbamylent à au moins environ 90 %, et où l'érythropoïétine est mise en contact avec le cyanate en présence de borate de potassium.

75. Procédé de production d'une protéine érythropoïétine carbamylée ayant moins d'environ 40 % de protéine agrégée et moins d'environ 40 % en masse de protéine sur- et sous-carbamylée, ce procédé comprenant la mise en contact d'une quantité d'érythropoïétine avec une quantité de cyanate à une température de 30°C à 34°C, à un pH et pendant un intervalle de temps suffisants pour que les groupes amine des lysines et des aminoacides N-terminaux de l'érythropoïétine se carbamylent à au moins environ 90 %.

76. Procédé de production d'une protéine érythropoïétine carbamylée ayant moins d'environ 40 % de protéine agrégée et moins d'environ 40 % en masse de protéine sur- et sous-carbamylée, ce procédé comprenant la mise en contact d'une quantité d'érythropoïétine avec une quantité de cyanate à une température, un pH et pendant un intervalle de temps suffisants pour que les groupes amine des lysines et des aminoacides N-terminaux de l'érythropoïétine se carbamylent à au moins environ 90 %, et où la protéine érythropoïétine carbamylée est purifiée à l'aide d'une chromatographie par échange d'anions en maintenant le pH du tampon d'écoulement et du tampon d'élution à 8,5 ± 0,2.
